(19) Europäisches Patentamt
European Patent Office
Office européen des brevets

(11) **EP 2 036 584 B1**

(12) **EUROPEAN PATENT SPECIFICATION**

(45) Date of publication and mention
of the grant of the patent:
**21.01.2015 Bulletin 2015/04**

(51) Int Cl.:
**A61M 1/36** *(2006.01)*          **B01J 20/24** *(2006.01)*
**C07K 19/00** *(2006.01)*          **C12N 15/62** *(2006.01)*

(21) Application number: **07767680.7**

(22) Date of filing: **27.06.2007**

(86) International application number:
**PCT/JP2007/062878**

(87) International publication number:
**WO 2008/001802 (03.01.2008 Gazette 2008/01)**

(54) **SUBSTRATE FOR BIOLOGICAL FLUID TREATMENT**

**SUBSTRAT ZUR BEHANDLUNG VON BIOLOGISCHEN FLÜSSIGKEITEN**

**SUBSTRAT POUR TRAITEMENT DE FLUIDE BIOLOGIQUE**

(84) Designated Contracting States:
**DE GB**

(30) Priority: **27.06.2006 JP 2006176487**

(43) Date of publication of application:
**18.03.2009 Bulletin 2009/12**

(73) Proprietor: **Asahi Kasei Kabushiki Kaisha
Osaka-shi
Osaka 530-8205 (JP)**

(72) Inventors:
• **SOHKA, Takayuki**
  **Tokyo 100-8440 (JP)**
• **NOMURA, Masayuki**
  **Tokyo 100-8440 (JP)**
• **MAEDA, Takuro**
  **Tokyo 100-8440 (JP)**
• **SUGIYAMA, Yuya**
  **Tokyo 100-8440 (JP)**

(74) Representative: **Von Kreisler Selting Werner -
Partnerschaft
von Patentanwälten und Rechtsanwälten mbB
Deichmannhaus am Dom
Bahnhofsvorplatz 1
50667 Köln (DE)**

(56) References cited:
WO-A1-00/75333          WO-A1-99/25463
WO-A2-2004/073864       JP-A- 2000 501 949
JP-A- 2003 501 114       JP-A- 2006 508 336
US-A1- 2004 258 698      US-A1- 2005 014 196

• DUBEL S ET AL: "Bifunctional and multimeric complexes of streptavidin fused to single chain antibodies (scFv)", JOURNAL OF IMMUNOLOGICAL METHODS, ELSEVIER SCIENCE PUBLISHERS B.V.,AMSTERDAM, NL, vol. 178, no. 2, 27 January 1995 (1995-01-27), pages 201-209, XP004021113, ISSN: 0022-1759, DOI: 10.1016/0022-1759(94)00257-W

• KIPRIYANOV SERGEY M ET AL: "Affinity enhancement of a recombinant antibody: Formation of complexes with multiple valency by a single-chain Fv fragment-core streptavidin fusion", PROTEIN ENGINEERING, OXFORD UNIVERSITY PRESS, SURREY, GB, vol. 9, no. 2, 1 January 1996 (1996-01-01) , pages 203-211, XP002149410, ISSN: 0269-2139

• TONYE LIBYH M ET AL: "A recombinant human scFv anti-Rh(D) antibody with multiple valences using a C-terminal fragment of C4-binding protein", BLOOD, vol. 90, no. 10, 15 November 1997 (1997-11-15), pages 3978-3983, XP55004421, ISSN: 0006-4971

• KOO K ET AL: "Development of a streptavidin-conjugated single-chain antibody that binds Bacillus cereus spores.", APPLIED AND ENVIRONMENTAL MICROBIOLOGY JUL 1998 LNKD- PUBMED:9647821, vol. 64, no. 7, July 1998 (1998-07), pages 2497-2502, XP002322628, ISSN: 0099-2240

- ERIC A. GROVENDER ET AL: "Single-chain antibody fragment-based adsorbent for the extracorporeal removal of beta2-microglobulin", KIDNEY INTERNATIONAL, vol. 65, no. 1, 1 January 2004 (2004-01-01), pages 310-322, XP55004456, ISSN: 0085-2538, DOI: 10.1111/j. 1523-1755.2004.00377.x

**Description**

Technical Field

The present invention relates to a substrate for biological fluid treatment for specifically adsorbing or removing a target of separation from a biological fluid such as blood, and the use thereof.

Background Art

Blood purification therapy is a therapy for the treatment of specified diseases, in which a substance component being a presumed related factor for the onset, induction, and/or exacerbation, is removed from the blood, so as to thereby achieve the maintenance of homeostasis *in vivo*, and is also referred to as apheresis therapy. Currently conducted blood purification therapy can be classified into four major categories, according to its target component of removal, and removal mechanism.

(1) Hemodialysis/hemofiltration/haemodiafiltration therapy:

This therapy is applicable to acute renal failure, chronic renal failure, and the like. The therapy is to directly purify the blood by removing components that should be discharged from the body in a form of urine (low molecular weight components such as urea and nitrogen). The main target is low molecular weight components in blood.

(2) Plasma exchange therapy:

This therapy is applicable to fulminant hepatitis, acute hepatic failure, arteriosclerosia obliterans, myasthenia gravis, Guillain-Barre syndrome, multiple sclerosis, myeloma, drug poisoning, pemphigus, and the like. The therapy is to separate and remove components that have been accumulated or produced in the body, from the blood. The main target is slightly large components such as cholesterol and immunoglobulin in blood.
It can be understood that the difference between plasma exchange and hemodialysis/hemofiltration/haemodiafiltration is the size of the substance to be removed from the blood. That is to say, hemodialysis is to remove small molecular weight substances whose molecular weight is up to several thousands Daltons, and hemofiltration is to remove medium-sized molecular weight substances whose molecular weight is not more than 20 to 30 thousands Daltons, while plasma exchange is to remove proteins or protein-conjugated harmful matters which have larger molecular weight and reside in blood (antibody, inflammatory cytokine, immune complex, liquid factor such as poison material, etc.).

(3) Hemoadsorption:

This therapy is applicable to septic shock due to endotoxin or Gram-negative bacterial infection, familial hyperlipemia, arteriosclerosia obliterans, focal glomerular sclerosis, dialytic amyloidosis, and the like. The therapy is to remove etiologic (related) substances through physico-chemical phenomenon by contacting blood or plasma with an adsorbent. The target of removal is plasma components such as endotoxin, LDL, and $\beta$2-microglobulin. A representative example of the adsorbent used herein is activated charcoal. In addition, there is a known adsorbent which adsorbs endotoxin by immobilizing polymyxin B that is capable of binding to an active center of endotoxin, lipid A, and neutralizing the activity thereof.

(4) Cytapheresis (Leukocytapheresis):

Although it is a type of hemoadsorption, different classification is applied because the target of removal is blood cell components. This therapy is applicable to ulcerative colitis, chronic rheumatoid arthritis, multiple sclerosis, dermatosis, immunological disease, and the like. The therapy is to remove cells, in particular leukocytes, serving as an etiologic (related) substance, through physico-chemical phenomenon or immune reaction by contacting blood with a substrate. Currently used substrates can achieve selectivity through their physico-chemical properties, examples of which include those made of cellulose acetate beads to have a selective adsorption property for granulocytes or monocytes, and those in a surface state having a selective adsorption property for activated leukocytes or platelets.
Recently, in such cytapheresis, studies have been conducted for improving the safety and therapeutic effect as well as reducing side effects by specifically capturing cells which is the target substance being a theoretically presumed etiology, through biological interaction such as immune reaction. Further, such techniques of blood cell treatment can be applied to the field of stem cell collection/transfusion in regenerative medicine, and thus are expected to cover general medical techniques for separating and treating blood cell components.

For the purpose of capturing cells which is the target substance with improved selectivity and specificity, Patent Document 1 has reported a separating material formed by immobilizing peptide or lipid having an affinity with T lymphocytes; which is, however, practically problematic since at least the exemplified binding affinity for T lymphocytes resulting from immobilization of thymic hormone is insufficient. In addition, Patent Document 2 through Patent Document 5 have respectively disclosed CD4 positive cell capturing materials formed by immobilizing peptide having an affinity with CD4 positive cells onto a nonwoven fabric; which are, however, practically inapplicable since the binding affinity for CD4 positive cells is insufficient only by immobilizing the exemplified peptide of complementarity determining region of heavy chain or light chain variable region of antibody. Further, Patent Document 6 has disclosed a separator for CD4-positive cells using a chimeric antibody, a single-chain antibody, or an antibody of a combination thereof binding to a CD4 molecule; which is, however, very difficult to apply to practical treatments since there is not described a method for removing CD4 positive cells with excellent reproducibility not from a monocyte suspension but from the whole blood. Patent Document 7 has disclosed an absorbent formed by immobilizing a ligand that has an affinity with a material expressed on a surface of an oncocyte to a high molecular weight compound through chemical bond; in which, however, the specificity of the exemplified ligand for the oncocyte is insufficient and no prerequisite for selectively and specifically capturing the target cell from the whole blood has been disclosed nor performed. Patent Document 8 has disclosed an immune cell adsorption material formed by immobilizing a material having an affinity for T helper type 2 surface antigen onto a water-insoluble carrier; in which, however, the adsorption rate is insufficient and no prerequisite for selectively and specifically capturing the target cell from the whole blood has been disclosed nor performed.

Incidentally, Non-patent Document 1 has disclosed an example in which a target substance was separated from a non-blood biological fluid. Firstly, a fusion protein having streptavidin at the end of a single-chain antibody against bacterial spore was prepared as a recognition molecule. Then, magnetic beads bound with anti-mouse antibodies as protein were previously prepared, and a chemically biotinylated substrate with respect to the amino groups of these antibodies was procured. The single-chain antibody-streptavidin fusion protein being the recognition molecule was contacted with the biotinylated substrate surface that had been produced in the above manner, to thereby produce magnetic beads immobilized with the single-chain antibodies over the substrate surface. Separation of bacterial spores from a biological fluid using thus produced magnetic beads is exemplified. Streptavidin is known to have a binding ability for low molecular biotin, and generally tend to form a tetramer. However, the binding form between biotin and streptavidin is absolutely non-covalent, and thus involves a concern of elution of the recognition molecule in practical use as a separating material. Furthermore, unless biotin is immobilized onto the substrate surface via a spacer with an appropriate degree of freedom in advance, binding is not achieved between the biotin that has been introduced into the substrate surface and the biotin-binding site in the single-chain antibody-streptavidin fusion protein being associated into a tetramer or higher order polymers. Therefore, this method requires complicated manipulations, and is practically insufficient in terms of stable immobilization of the target substance onto the substrate surface.

Non-patent Document 1 uses commercially available magnetic beads which was bound with sheep anti-mouse antibodies as a substrate for biotin-mediated immobilization of recognition molecules. Biotin having its carboxy group activated by N-hydroxysuccinimide is chemically bound to the amino group of the sheep anti-mouse antibodies on the surface of these magnetic beads. This document also presents an experimental data obtained by immobilization of the recognition molecules only by means of physical adsorption without chemical biotinylation, and mentions that such immobilization is quite inferior to biotin-mediated immobilization in the separation ability for bacterial spores. Further, in this case, it is shown that detachment easily occurs by washing. The magnetic beads used herein had been already immobilized with sheep anti-mouse antibodies on the surface. Accordingly, in order to covalently immobilize the recognition molecule likewise of the present application, active groups for immobilization have to be reintroduced into the bead surface. It means, for example, that carboxyl groups derived from sheep anti-mouse antibodies which already exist on the surface, have to be activated with N-hydroxysuccinimide, or that hydroxyl groups derived from such antibodies have to be activated with epichlorohydrin (chloromethyloxirane); which is, however, very difficult and inefficient. Even if small amounts of active groups can be successfully introduced, the subsequent immobilization of the recognition molecule is considered to mainly account for physical adsorption. As a result, similarly to the above, such immobilization is predictably quite inferior to biotin-mediated immobilization in the separation ability for bacterial spores. Further, it is possible to activate carboxyl groups on the recognition molecule side to be immobilized, with N-hydroxysuccinimide, or to covalently bind the recognition molecule and sheep anti-mouse antibody-derived amino groups on the magnetic beads where sheep anti-mouse antibodies were immobilized on the surface, with carbodiimide etc; in which case, however, intramolecular or intermolecular cross linkage occurs between recognition molecules, and thus the method is inefficient.

In addition, since those skilled in the art would achieve an idea to efficiently use the ligand site of the recognition molecule in an immobilized state, they would consider that it is inefficient to site-unspecifically and covalently immobilize molecules of an assembly structure in which four or more recognition molecules are associated through their association domains, in consideration of deactivation of the ligand site due to the covalent bond. That is, the idea exemplified in this application is very difficult to conceive for those skilled in the art, in which the separation ability for cells or substances is improved by having the molecular form of the recognition molecule in a covalently immobilized state on the substrate surface.

In addition, fusion proteins having streptavidin at the end of single-chain antibodies as mentioned above are publicly known as targeting molecules for the radiotherapy cancer treatment (Patent Document 9, Patent Document 10, Non-patent Document 2, and Non-patent Document 3). Their usage application basically aims for effective cancer treatment through administration of the fusion protein into the body, and accumulation thereof at the cancer site, followed by administration of radioactive labeled biotin derivative, in which neither an example nor an implying description can be found which shows that the separation of target substance by means of immobilization of the fusion protein onto the substrate surface. Therefore, their technical idea is different from that of the present invention. Further, it is reported that, since fusion proteins having streptavidin at the end of single-chain antibodies form a tetramer or higher order polymer as mentioned above, then the binding affinity thereof for the target molecule is higher than that of monomer single-chain antibodies (Non-patent Documents 4 and 5). However, all of these reported papers merely discuss the affinity when the fusion protein having streptavidin at the end of a single-chain antibody is not immobilized, and neither an example nor an implying description can be found which shows that the separation of target substance by means of immobilization of the fusion protein onto the substrate surface. Therefore, their technical idea is different from that of the present invention.

Moreover, Patent Document 11 has disclosed a molecule of a structure resembling an associated assembly structure disclosed in this application, comprising four or more recognition molecules. However, the above disclosure is for use in the treatment or diagnosis against diseases by means of administration into human, and is not for the purpose of separating the target substance while immobilizing the recognition molecule likewise of the present invention. The technical idea thereof is also different.

Non-patent Document 6 has disclosed a method for separating *Listeria monocytogenes* using magnetic beads in which single-chain antibodies for *Listeria monocytogenes* that are biotinylated at their ends, are immobilized onto streptavidin-conjugated magnetic beads. This is one of the directional immobilization methods of the ligand site to the substrate surface that can be easily conceived by those skilled in the art. However, there is a problem in that the separation efficiency for *Listeria monocytogenes*, that is, the target substance, is insufficient, and the practical use thereof is difficult, regardless of its impurity-free experimental system.

As to the recognition molecule having a specific binding affinity for the target substance, known examples include an antibody, a chimeric antibody, a single-chain antibody, F(ab')$_2$, Fab, Fab', a peptide, a nucleic acid, a sugar chain, and a low molecular weight compound. Generally, antibodies have high binding affinities and readily retain their binding activities even if immobilized onto a substrate, while having a problem in terms of practical application because of the high production cost. On the other hand, antibody derivatives, represented by single-chain antibodies made from antibodies by lowering their molecular weight through genetic engineering procedures, have a merit of inexpensive production cost as compared to antibodies. However, it is difficult for such antibody derivatives to retain practically sufficient binding activities in an immobilized state onto the substrate surface.

In this way, although it has been expected to specifically capture the target substance in a biochemical fluid such as blood, in particular to directly capture cells from the whole blood, no practically applicable technique has been established yet.

Patent Document 1: Japanese Unexamined Patent Application, Publication No. Hei 3-32680

Patent Document 2: Japanese Unexamined Patent Application, Publication No. Hei 6-154316

Patent Document 3: Japanese Unexamined Patent Application, Publication No. Hei 6-154317

Patent Document 4: Japanese Unexamined Patent Application, Publication No. Hei 6-218051

Patent Document 5: Japanese Unexamined Patent Application, Publication No. Hei 6-269663

Patent Document 6: Japanese Unexamined Patent Application, Publication No. Hei 11-332594

Patent Document 7: Japanese Unexamined Patent Application, Publication No. 2000-217909

Patent Document 8: Japanese Unexamined Patent Application, Publication No. 2003-52817

Patent Document 9: Japanese Translation of PCT International Application, Publication No.2003-501096

Patent Document 10: PCT International Publication No. WO00/075333

Patent Document 11: PCT International Publication No. WO2006/107617

Non-patent Document 1: Applied and Environmental Microbiology, July, 1998, pp. 2497-2502

Non-patent Document 2: Blood 2004, 104, pp. 227-236

Non-patent Document 3: Clinical Cancer Research, 2000, vol.6, pp.406-414

Non-patent Document 4: Protein Engineering, 1996, vol. 9(2), pp.203-211

Non-patent Document 5: Human Antibodies Hybridomas, 1995, 6(3), pp.93-101

Non-patent Document 6: Foodborne Pathogens and Disease, 2007, 4(1), pp.74-83

Dubel et al. (1995) J. Immunol. Meth. 178, 201-209, discloses the preparation of a fusion protein comprising a single chain antibody (scFv), a core-streptavidin, a c-myc tag, an extra free cysteine, and a His-tag These fusion proteins assemble into tetramers. The fusion protein can be extracted from inclusion bodies using denaturation and renaturation steps. The fusion proteins can be used for in-vitro purging of autologous bone-marrow, and for the preparation of affinity columns for the binding of ligands to the scF-v moieties.

Grovender et al. (2004) Kidney Int. 65, 310-322, and WO 99/25463 disclose the provision of hemodialysis devices comprising a substrate comprising a scFv that is covalently bound to a solid support for the removal of a substance from whole blood.

Disclosure of the Invention

Object to be Solved by the Invention

Not only in cytapheresis, but also in general medical techniques for separating and treating blood cell components, it has been expected to improve the safety and therapeutic effect as well as reducing side effects by selectively and specifically capturing cells which is the target substance being a theoretically presumed etiology, through biological interaction such as immune reaction. However, there is a problem that no practically applicable technique which specifically captures cells which is the target substance in blood, in particular directly from the whole blood, has been established. Moreover, separation of the target substance from a non-blood biological fluid involves a problem in that complicated and expensive manipulations are required for previously immobilizing biotin onto the substrate surface and then non-covalently immobilizing the target molecule through the affinity of biotin-streptavidin, and a problem in that it is difficult to immobilize the ligand moiety in an effective form to recognize the target substance.

It is an object of the present invention to provide a practically applicable substrate for blood treatment for specifically capturing a target substance such as a cell directly from the whole blood, and an inexpensively and industrially applicable substrate for biological fluid treatment which is capable of separating a target substance directly from a biological fluid. Another object of the present invention is to provide a device and a separation method using this substrate.

Means for Solving the Object

In view of the above problems, the inventors of the present invention have conducted intensive studies. Firstly, they have deeply considered the validity of the result that can be easily conceived by those skilled in the art, in which the binding activity can be exerted even in an immobilized state if a large amount of a derivative, made from an antibody whose molecular weight has been lowered by genetic engineering procedures, is supplied as a recognition molecule to immobilize onto the substrate surface. The above result implies that the majority of the recognition molecule immobilized onto the substrate surface is in a form lacking the binding activity if it is in an immobilized state.

Therefore, the inventors of the present invention have made an attempt for directional immobilization in which a domain for immobilization onto the substrate surface was attached to the ligand moiety in the recognition molecule which selectively binds to the target substance, so that the target molecule could be immobilized onto the substrate surface via this association domain. Thereupon, however, an amazing result which had been totally unpredictable from the first hypothesis of directional immobilization was discovered; in which, in reality, the binding activity for the target molecule was remarkably retained by covalently immobilizing the recognition molecules in an assembled state through their association domains having a self-association property, onto the substrate surface while including as little non-covalent bond such as biotin-streptavidin as possible, rather than directional immobilization of the association domain on the substrate side. This has led to the completion of the present invention.

Thus, the present invention provides the following invention.

(1) A substrate for biological fluid treatment in which an assembly of recognition molecules having a selective binding property for at least one target substance which is selected from a biorelated substance, a bacterium, a cell, a cell aggregation, a virus, or a virus-infected cell, is covalently immobilized on a surface of the substrate,
wherein each recognition molecule is a linear molecule comprising a ligand moiety which selectively binds to the target substance, and an association domain,
wherein four recognition molecules form the assembly structure through association of their association domains,
wherein the assembly is covalently bound to the substrate via the ligand moieties of the recognition molecules in such a way that at least one ligand moiety remains externally oriented with respect to the substrate surface, wherein each recognition molecule is a single-chain polypeptide obtained by expression of a nucleic acid in which a nucleic acid that encodes the ligand moiety for constituting the recognition molecule and a nucleic acid that encodes the association domain are ligated, and
wherein the association domain is a polypeptide region translated from a nucleic acid that encodes a full length, or a part of, streptavidin or avidin.

(2) The substrate for biological fluid treatment according to (1), wherein the assembly of recognition molecules is directly bound to the substrate surface or via active groups.

(3) The substrate for biological fluid treatment according to (2), wherein the active group is an epoxy group obtained through activation of hydroxyl groups and amino groups on the substrate surface using epichlorhydrin.

(4) The substrate for biological fluid treatment according to (2), wherein the active group is introduced into the substrate surface by means of graft polymerization, plasma treatment, corona treatment, chemical treatment, or exposure to gas.

(5) The substrate according to (1), wherein the polypeptide is obtained by expression of a nucleic acid in which a nucleic acid that encodes the ligand moiety for constituting the recognition molecule and a nucleic acid that encodes the association domain are ligated by a nucleic acid that encodes a spacer, and the spacer is a peptidic spacer composed of 0 to 100 amino acids.

(6) the substrate for biological fluid treatment according to (1) or (5), wherein the polypeptide to be translated from the nucleic acid that encodes the ligand moiety has a molecular weight of 100 kD or less.

(7) The substrate for biological fluid treatment according to any one of 1, 5 or 6, wherein the polypeptide to be translated from the nucleic acid that encodes the association domain has a molecular weight of 50 kD or less.

(8) The substrate for biological fluid treatment according to any one of 1 or 5 to 4, wherein the polypeptide to be translated from the nucleic acid that encodes the ligand moiety is a single-chain antibody.

(9) The substrate for biological fluid treatment according to any one of 1, or 5 to 8, wherein the molecular weight ratio of the polypeptide that constitutes the ligand moiety to the polypeptide that constitutes the association domain is within a range of 1:10 to 20:1.

(10) The substrate for biological fluid treatment according to any one of 1 or 5 to 9, which is produced by:

- translating a nucleic acid that encodes a recognition molecule having a selective binding property for the target substance, into a polypeptide;
- denaturing/solubilizing the recognition molecule polypeptide that has been insolubilized during the process of said translation, with an aqueous solution containing guanidine hydrochloride or urea; and
- subsequently renaturating the three-dimensional structure of the recognition molecule polypeptide by removing guanidine hydrochloride or urea.

(11) The substrate for biological fluid treatment according to any one of 1 or 5 to 10, which is produced by:

- introducing an active group for forming a covalent bond with a recognition molecule having a selective binding property for the target substance into a surface of a water-insoluble substrate; and
- covalently immobilizing the recognition molecule onto the substrate surface by contacting the water-insoluble substrate with an aqueous solution containing the recognition molecule at a temperature of 0°C to 50°C for 10 or more seconds.

(12) A method for production of the substrate for biological fluid treatment according to any one of 1 or 5 to 11, which comprises:

(i) a step of translating a nucleic acid that encodes a recognition molecule having a selective binding property for at least one target substance which is selected from a biorelated substance, a bacterium, a cell, a cell aggregation, a virus, or a virus-infected cell, into a polypeptide;
(ii) a step of denaturing/solubilizing the recognition molecule polypeptide that has been insolubilized during the process of said translation, with an aqueous solution containing guanidine hydrochloride or urea; and
(iii) a step of, after the step (ii), renaturating the three-dimensional structure of the recognition molecule polypeptide by removing guanidine hydrochloride or urea.

(13) A method for production of the substrate for biological fluid treatment according to any one of 1 or 5 to 12, which comprises :

(i) a step of introducing an active group into a surface of a water-insoluble substrate for forming a covalent bond with a recognition molecule having a selective binding property for at least one target substance which is selected from a biorelated substance, a bacterium, a cell, a cell aggregation, a virus, or a virus-infected cell; and
(ii) a step of covalently immobilizing the recognition molecule onto the substrate surface by contacting the water-insoluble substrate with an aqueous solution containing the recognition molecule at a temperature of 0°C to 50°C for 10 or more seconds.

(14) A device for biological fluid treatment which is obtained by loading the substrate for biological fluid treatment according to any one of 1 to 11 in a container having an inlet and an outlet.

(15) The divice for biological fluid treatment according to 14 which is used for the treatment of a disease.

(16) A method for production of the device for biological fluid treatment according to (14) or (15), which comprises a step of loading the substrate for biological fluid treatment according to any one of (1) to (11) in a container having an inlet and an outlet.

(17) A method for treatment of a biological fluid, which comprises a step of flowing an aqueous solution containing a target substance through the device for biological fluid treatment according to (14) or (15), wherein methods for the treatment of the human or animal body by surgery or therapy are excluded.

(18) A method for treatment of blood, which comprises a step of flowing blood containing a target substance through the device for biological fluid treatment according to (14) or (15), wherein methods for the treatment of the human or animal body by surgery or therapy are excluded.

Best Mode for Carrying Out the Invention

The term "target substance" used herein means a biologically derived matter specified as a target of removal, among biologically derived substances, cells, and tissues contained in a biological fluid such as blood, and is at least one substance selected from a biorelated substance, a bacterium, a cell, a cell aggregation, a virus, or a virus-infected cell. The target substance may not only be contained in blood itself, but may also be contained in a blood-derived liquid having lots of impurities.
The term "biorelated substance" among the target substances refers to a biologically produced protein, nucleic acid, lipid, saccharide, low molecular chemical compound, or the like. Further, the term "biorelated substance" used herein can include even an artificial matter such as an endocrine disrupter which seriously affects living bodies. The biorelated substance also includes harmful matters. The term "harmful matter" means, for example, LDL, $\beta 2$ microglobulin, drugs, bilirubin, bile acid, amino acids, creatinine, endotoxin, an anti-A antibody, an anti-B antibody, an anti-acetylcholine receptor-antibody, IgG, anti-cardiolipin antibody, anti-DNA antibody, immune complexes, coma-inducing substances, rheumatoid factors, and other plasma components. Abnormal prions are also included therein. In addition, various mediators such as inflammatory cytokines can also serve as harmful matters depending on the pathologic condition. As to inflammatory cytokines, IL-1, IL-6, IL-8, IL-18, TNF-$\alpha$, GM-CSF, and RANTES/SIS cytokine families are known. Moreover, mediators involved in inflammation include prostaglandin, thromboxane, leukotriene and other products which is produced from arachidonic acid *in vivo*, platelet activating factors, histamine, bradykinin, and a complement factor C5a.
As to the bacterium, any type of bacterium may be enumerated without specific limitations. For example, most bacteria are spherical, rod-shaped, or spiral-shaped, in the size of about 0.5 to 4 $\mu$m. The outermost layer of the cell consists of a thick cell wall, which encloses a thin cell membrane inside. In addition, some bacteria are capsulated, flagellated, or fimbriated, while other bacteria form spores depending on the environmental variation. Bacteria can be classified into Gram positive and Gram negative on the basis of the staining affinity; however, this classification is not to be considered as limiting. In addition, bacteria can also be classified into autotrophs and heterotrophs, or aerobes and anaerobes; however, these classifications are not to be considered as limiting, either.
Biologically, archaebacteria are microorganisms of totally different species from bacteria; however, they are exemplified as bacteria in this application. Archaebacteria can include thermophiles, extreme halophiles, methanogens, and so forth.
As to the cell, any type of cell may be enumerated without specific limitations, and examples thereof can include pathogenic cells. The term "pathogenic cell" refers to a presumed causative factor of a specified disease, among tumor cells, bacteria, or blood cells such as leukocytes (neutrophils, eosinophils, basophils, lymphocytes, or monocytes) and platelets. For example, when enhanced cellular immunity is concerned in autoimmune diseases, pathogenic cells are desirably distinguished on the basis of immunoglobulin superfamily molecules expressed on the surface of leukocyte cells. Specific examples thereof include CD2 positive cells, CD4 positive cells, CD8 positive cells, CD28 positive cells, CTLA-4 (CD152) positive cells, PECAM-1 (CD31) positive cells, ICAM-1 (CD54) positive cells, and ICAM-2 (CD102) positive cells. In addition, it is also effective to distinguish on the basis of integrin family molecules. Examples thereof include, but not limited to, VLA-1 (CD49a/CD29) positive cells, VLA-2 (CD49b/CD29) positive cells, VLA-3 (CD49c/CD29) positive cells, VLA-4 (CD49d/CD29) positive cells, VLA-5 (CD49e/CD29) positive cells, VLA-6 (CD49f/CD29) positive cells, LFA-1 (CD11a/CD18) positive cells, Mac-1 (CD11b/CD18) positive cells, p150, 95 (CD11c/CD18) positive cells, gpIIb/IIIa (CD41/CD61) positive cells, and LPAM-1 positive cells.

The term "cell aggregation" refers to a plurality of cells in an assembled state. Examples thereof include, but not limited to, a pancreatic islet of Langerhans.

As to the virus, any type of virus may be enumerated without specific limitations. For example, most viruses have their sizes of about 20 to 250 nm (nanometer: one millionth of a millimeter). The difference from bacteria is that they are unable to proliferate without parasitism (infection) in a living host cell. For the sake of explanation, main viruses infectious to animals are exemplified as follows; however, these are not to be considered as limiting. Parvovirus, papillomavirus, polyoma virus, adenovirus, hepadnavirus, herpesvirus, varicella-zoster virus, cytomegalovirus, EB virus, poxvirus, smallpox virus, vaccinia virus, reovirus, rotavirus, calicivirus, norovirus, picornavirus, poliovirus, enterovirus 71, coxsackie virus, echovirus, rhinovirus, hepatitis A virus, astrovirus, Togavirus, rubella virus, flavivirus, Japanese encephalitis virus, West Nile virus, tick-borne encephalitis virus, dengue virus, hepatitis C virus, human immunodeficiency virus, coronavirus, filovirus, Ebola virus, rhabdovirus, rabies virus, bunyavirus, hantavirus, orthomyxovirus, influenza virus, paramyxovirus, measles virus, mumps virus, arenavirus, and Lassa virus. Moreover, viruses infectious to plants and bacteria are also included.

The term "virus-infected cell" refers to a cell or bacterium infected with the aforementioned or any another virus. Those in a preinfected state where any virus is bound to a receptor on the cellular surface, are also included. Inside the virus-infected cell, fresh viruses are produced through gene replication and protein synthesis of the virus. In addition, even in cases where any virus-derived nucleic acid is recombined into the genome of a host cell and virus particles are not produced, the host cell can be regarded as the virus-infected cell.

The term "biological fluid" used herein refers to a fluid produced from a living body, such as sweat, blood, lymph fluid, spinal fluid, urine, tissue fluid, saliva, mucus, semen, secretion, or digestive fluid, as well as a culture liquid, lysate, or extract of cells, bacteria, or viruses, a fluid resulting from mechanical fragmentation of an organ, a digestive fluid of an enzyme-treated organ, and the like. Not only these straight solutions, but also solutions diluted with a buffer or organic solvent, are also included.

The term "substrate" used herein refers to a material in a solid state in an aqueous solution at normal temperature. The shape thereof is not specifically limited, although the surface area is preferably larger so as to increase the frequency of contact with target cells in the liquid phase. Examples of the shape can include spherical form, cubic form, planar form, flat film form, chip form, fibrous form, floc form, cloth form, thread form, bundle form, woven fabric form, nonwoven fabric form, and other fiber structures, polymeric porous form such as sponge, bead form, gel form, and hollow fiber form. On these, spherical form, granular form, and fibrous form are desirable in terms of the easiness of close packing, the easiness of even retainment of recognition molecules on the surface, the capability of having a relatively large effective area, and the circulation of a biological fluid such as blood. In particular, when cells in blood are to be separated or adsorbed, woven fabric form and nonwoven fabric form are preferable in terms of the adsorption efficiency. Further, nonwoven fabric form is most preferable in terms of the easiness of structural control of the carrier.

A variety of intermolecular forces are working between the recognition molecules and the substrate surface. Physical adsorptive power and attracting force to immobilize these molecules onto the substrate surface are considered to be included. The term "covalently" used herein refers to a state where the majority of molecules constituting the recognition molecules form covalent bonds either directly, or via functional groups, with the substrate surface, in the binding between the recognition molecules and the substrate surface. This can achieve the immobilization in which detachment hardly occurs even by vigorous washing with a surfactant.

As to the method for confirming that "the majority of molecules constituting the recognition molecules are covalently bound to the substrate surface", there can be used a 6M guanidine-HCl aqueous solution and an 8M urea aqueous solution which have a strong protein denaturating effect and are capable of solubilizing proteins. That is to say, after immobilization and washing, the process of denaturation/solubilization for the recognition molecules on the substrate surface with the abovementioned strong denaturing agent enables detachment of many recognition molecules bound to the substrate surface by noncovalent intermolecular forces such as physical adsorption. The binding between streptavidin and biotin is a noncovalent biochemical bond and is considered to be $Kd = 10^{-15}$ M at the strongest. The above method is effective for such a binding manner since the streptavidin protein is denatured to lose its binding ability. Thereafter, by detecting/quantifying (for example, SDS-PAGE analysis) thus detached recognition molecules, or by detecting/quantifying (for example, total protein quantification or elemental analysis) such recognition molecules remaining on the substrate itself that has been treated with the abovementioned denaturing agent after washing well with water, it becomes possible to confirm that the recognition molecules have been covalently introduced into the substrate surface. In this case, the pH of the solution having the denaturing agent is desirably around neutral at about pH = 4 to 9, and should not be extremely acidic nor alkaline. This is because that the recognition molecules may be subjected to hydrolysis reaction, which may cause decomposition and detachment of these recognition molecules regardless of covalent immobilization thereof. In addition, heat can also be applied at the time of the treatment with the denaturing agent; in which case, decomposition of the recognition molecules may also occur, and therefore the interpretation of results should be carefully carried out. Further, although it is possible to denature the recognition molecules with an organic solvent such as phenol, the recognition molecules may be insolubilized with water to show pseudo-positive; in which case, the interpretation of results should also be carefully carried out.

The material of the substrate may be either an inorganic compound or an organic compound as long as it is capable of stably immobilizing at least a predetermined amount of the recognition molecule, although it is preferably a safe material for use as a medical material which can be processed into various forms, which is capable of chemically binding to the recognition molecule either directly or indirectly, and which produces little eluted matter when being contacted with a biological fluid such as blood. Examples thereof can include inorganic materials such as glass, kaolinite, or bentonite, polysaccharide-based compounds of plant origin such as cellulose, natural polymers such as dextran, chitin, chitosan, starch, agarose, protein (collagen etc.), or natural rubber, polypropylene, polystyrene, polyamide, polyethylene, polyurethane, polyvinyl alcohol, ethylene-vinylalcohol copolymer, polymers and copolymers of polymethacrylic ester, polyacrylic ester, polyacrylic acid, polyvinyl alcohol, other vinyl-based compounds, or derivatives thereof, polyamide-based compounds such as Nylon 6 or Nylon 66, polyester-based compounds such as polyethylene terephthalate, synthetic polymers such as polyamino acids, and activated carbon.

When the selected substrate is in a nonwoven fabric form, the fiber constituting the nonwoven fabric substrate is preferably cellulose, polyester, or polypropylene, in terms of the strength, the processability, or the safety. In addition, the structure of such a nonwoven fabric substrate can be controlled by appropriately selecting the average diameter of constituent fibers. The average diameter of constituent fibers of this substrate is preferably 2.5 $\mu$m or more but less than 50 $\mu$m. This is because that fibers having an average diameter of less than 2.5 $\mu$m are unfavorable since they often result in fine texture when made into a nonwoven fabric form, which causes nonspecific cell adsorption in that non-target cells are physically captured. On the other hand, fibers having an average diameter of 50 $\mu$m or more are unfavorable since they often result in rough texture when made into a nonwoven fabric form, which causes remarkable reduction in the frequency of contact with target cells and therefore lowers the removal efficiency.

The term "recognition molecule" used herein refers to an artificial molecule which is produced with use of protein engineering and/or molecular biological, and genetic engineering techniques. More in detail, the term refers to a linear molecule comprising a ligand moiety which selectively binds to a target substance, and an association domain. In addition, a spacer may exist between the ligand moiety and the association domain.

The recognition molecule specifically used in the present invention is referred to above. The term "ligand moiety" used herein refers to a portion having a selective binding property for a target substance. It is selected from antibody's F(ab')$_2$ portion, Fab portion, and Fab' portion, peptides, gene recombinant proteins, nucleic acids, sugar chains, low molecular weight compounds, and so forth, although it is desirably a gene recombinant protein or a nucleic acid. Because it is in an artificial molecule, there is a merit in which those having excellent heat stability can be provided by mass production at low cost. Such a ligand may also be called "artificial antibody" because it specifically binds to a target. The artificial antibody is normally produced with use of a molecule having a similar structure to the tertiary structure of antibody V region, through randomization of the loop formation region, or incorporation of CDR3 of the antibody V gene. Examples thereof include: Affibody produced from Protein A to resemble an antibody (K. Nord, O. Nord, M. Uhlen, et al., Eur. J. Biochem, 268, 4269 (2001)); an illuminating antibody, Fluorobody, produced from GFP to resemble an antibody (I. S. Kim, J. H. Shim, Y. T. Suh, et al., Biosci. Biotechnol. Biochem., 66, 1148 (2002)); Minibody produced from fibronectin domain 10 to resemble an antibody (V. Batori, A. Koide, and S. Koide, Protein Eng., 15, 1015 (2002)); Pepbody which is a fusion molecule of a peptide mimic molecule and an antibody (E. Lunde, V. Lauvrak, I. B. Rasmussen, et al., Biochem. Soc. Trans., 30, 500 (2002)); and a single-chain antibody Nanobody which uses a structure of camel antibody (A. Muruganandam, J. Tanha, S. Narang, and D. Stanimirovic, The FASEB J. 16, 240 (2002)). In addition, the examples also include Aptamer having a nucleic acid structure, and Spiegelmer which uses an RNA optical isomer, enatio-RNA (B. Wlotzka, S. Leva, B. Eschgfaller, et al., Proc. Natl. Acad. Sci. USA, 99, 8898 (2002)).

The term "single-chain antibody" used herein refers to an antibody in a form of a single-chain polypeptide formed by linking a (heavy chain V region) VH domain (heavy chain V region) and a VL domain (light chain V region) which are important for the binding between the antibody molecule and an antigen, by a peptide called linker. In this application, those represented by Single-chain Fv or scFv are used in the same meaning for the single-chain antibody.

As to the size of the ligand moiety, a polypeptide having a molecular weight of 100 kD or less is desirable so as not to inhibit the self-association of "association domains" that will be described later.

The term "association domain" used herein refers to a portion which constitutes a lump three-dimensional structure of the recognition molecule but is devoid of the "ligand moiety", and which has a property of forming an associated assembly with other association domain(s). According to the invention the association domain is a polypeptide region translated from a nucleic acid that encloses a full length, or a part of avidin or streptavidin, an artificial peptide sequence for forming a dimmer in a helix-turn-helix structure (J Willuda et al., The Journal of Biological Chemistry, 276, 14385, (2001)), and a domain sequence for forming a p53 protein-derived tetramer (M Rheinnecker et al., The Journal of Immunology 157, 2989, (1996)). Avidin and streptavidin are used in terms of the ability of forming a tetramer, or higher order associated assembly. As to the size of this "association domain", a 1 kD polypeptide or greater is desirable in order to exert the ability of forming an associated assembly between association domains, and a 50 kD polypeptide or smaller is desirable in terms of the stability of the whole assembly made of recognition molecules. The term "spacer" used herein refers to a linear molecule covalently linking between the "association domain" and the "ligand moiety" in the recognition molecule, and preferably a peptidic molecule. It is a flexible portion the structure of which can not be clearly determined even by X-ray analysis, or NMR analysis, of the crystal structure. This spacer is preferably in a length of one or more amino acids so as not to inhibit the formation of respective three-dimensional structures of the "association domain" and the "ligand moiety". On the other hand, if the length is too long as the spacer, the stability as a protein is lowered. Therefore, the length is preferably 100 or less amino acids. More preferably usable length is about 3 to 50 amino acids. In addition, a function as a spacer can also be achieved by inserting a purification tag sequence thereinto in a form of a protein. Examples thereof can include, but not limited to, the FLAG tag sequence (DYKDDDDK) and the His tag sequence (HHHHHH). The molecular weights of polypeptides that constitute respective abovementioned portions and the length of the spacer are important for recognition molecules in the present invention to be self-associated into an assembly structure through their "association domains". In addition, in order to efficiently form such assemblies, the molecular weight ratio of the polypeptide that constitutes the "ligand moiety" to the polypeptide that constitutes the "association domain" is desirably within a range of 1:10 to 20:1, and more preferably 1:5 to 10:1.

The term "selective binding property" used in the term "recognition molecule having a selective binding property for a target substance" herein refers to a property of exclusively binding to a target substance with high binding affinity by means of biological interaction such as antibody-antigen binding. The dissociation constant between the immobilized recognition molecule and the target substances is preferably not higher than 1 $\mu$M, more preferably not higher than 10 nM, and yet more preferably not higher than 1 nM.

The term "dissociation constant" used herein is usually denoted $K_D$ which indicates the degree of dissociation. A smaller dissociation constant means more tight binding, which is an index commonly used to describe the strength of binding affinity by those skilled in the art. After target protein molecules at various concentrations have been sufficiently bound to recognition molecules, the dissociation constant can be calculated by quantification of free target protein molecules. Examples of the quantification method include Enzyme-Linked ImmunoSorbent Assay (ELISA) technique, spectroscopy technique, a method using a crystal oscillator, a method using Frontal Affinity Chromatography (M. Nishikata, K. Kasai, S. Ishii, J. Biochem. 82, 1475 (1977)), a method using RI-labeled target protein molecules, and a method using surface plasmon resonance phenomenon. Of these, for example, in cases of a biosensor using surface plasmon resonance phenomenon, a sensor chip composed of a treated glass surface deposited with a gold film, is used to immobilize recognition molecules, and then a solution containing target protein molecules is poured so as to bring contact between the recognition molecules and the target protein molecules. Light having a constant wavelength is irradiated onto the gold film before and after the contact, to observe the timewise changes in the reflected light. Since the reflected light is affected by the density of substances in the vicinity of the gold film due to the surface plasmon resonance phenomenon, real-time changes in the binding amount can be understood by the changes in the surface density, so that the binding rate constant and the dissociation rate constant can be obtained to thereby readily determine the dissociation constant.

The method for refolding the recognition molecule from a denatured/solubilized state in the present invention is not specifically limited, although a guanidine hydrochloride, urea, or the like can be used as a denaturing agent for solubilizing the recognition molecule. The renaturating action for the three-dimensional structure of a protein or nucleic acid molecule is generally referred to as refolding. Examples of methods preferably used for this refolding action include a method in which denatured/solubilized recognition molecules are rapidly diluted with a buffer free from a denaturing agent, and a method in which the concentration of the denaturing agent is lowered stepwise by means of dialysis. In addition, Japanese Unexamined Patent Application, Publication No. 2000-93172 has disclosed a method in which an oxidizing agent is added in order to promote the formation of disulfide bonds within proteins at a given guanidine hydrochloride concentration, and a method in which an antiaggregating agent such as L-arginine is added in order to suppress the aggregation of proteins during renaturation of the three-dimensional structure. These methods can also be quite suitably used for the present invention.

Regarding the immobilization of the recognition molecules onto the substrate surface, the recognition molecules may be bound to the substrate surface either directly, or via active groups. Any publicly known reaction can be used, and examples thereof can include substitution reaction, addition reaction, condensation reaction, polymerization reaction, and ring cleavage reaction.

When the recognition molecules are to be covalently bound to the substrate surface via active groups, the active group may be any publicly known functional group as long as its structure allows a covalent bond between the recognition molecule and the substrate. Examples thereof can include: $\alpha$-acetamino halogen groups obtained through activation of the substrate surface with use of N-hydroxymethylhaloacetamide, N-hydroxymethyldihaloacetamide, or the like; $\alpha,\beta$-propionamino halogen groups obtained through activation of the substrate surface with use of N-hydroxymethyldihalopropionamide or the like; $\alpha,\alpha$-acetamino dihalogen groups obtained through activation of the substrate surface with use of N-hydroxymethyldihaloacetamide or the like; $\alpha,\alpha,\alpha$-acetamino trihalogen groups obtained through activation of the substrate surface with use of N-hydroxymethyltrihaloacetamide or the like; and other active group obtained through activation of the substrate surface with use of a haloacetamino alkanating agent; or epoxy groups obtained through activation of functional groups, such as a hydroxyl group and an amino group, on the substrate surface with use of epichlorohydrin or the like. Other suitable examples of such active groups include, but not limited to, N-hydroxysuccinimide ester groups obtained through activation of carboxy groups with N-hydroxysuccinimide, an isocyanate group, an isothiocyanate group, an amino group, a carboxyl group, a hydroxyl group, a vinyl group, a maleimide group, a tosyl group, a tresyl group, or an active group obtained with use of cyanogen bromide. In terms of the ability to react under a mild condition while retaining the function of the recognition molecule, preferred are active groups obtained through activation of the substrate surface with use of a haloacetamino alkanating agent, epoxy groups, N-hydroxysuccinimide ester groups, and a maleimide group.

Further, as to the method for introducing an active group into the substrate surface, it is also suitable to introduce a functional group or an active group into the substrate surface by means of graft polymerization, plasma treatment, corona treatment, chemical treatment, or exposure to gas. If daringly exemplified, it is readily possible to produce a substrate introduced with an active group or a functional group, which can be used for the immobilization, by y ray irradiation onto polystyrene, polypropylene, polyethylene terephthalate, or the like, followed by immersion thereof into a vinyl-based monomer solution such as glycidyl methacrylate in a non-oxygen condition. These graft chains may be either prepared with use of single monomers, or in a copolymer form resulting from a mixture of a plurality of monomers. It is also absolutely possible to prepare active groups through introduction of a hydroxyl group, a carboxyl group, an amino group, or the like, by means of graft polymerization, followed by activation by the abovementioned method. Plasma treatment and corona treatment are known to be able to introduce various types of functional groups into the substrate surface. If daringly exemplified, it is possible to introduce a hydroxyl group or a carboxyl group into the surface of a polyethylene terephthalate substrate under the presence of oxygen. Such surface modifications can also be applied to the present invention. In addition, it is also possible to immobilize the recognition molecule through introduction of a photoreactive azo group or diazo group into the substrate surface, followed by irradiation with light or radiation under the presence of the recognition molecule.

Moreover, it is also possible to introduce the abovementioned active group into the substrate surface by coating a polymer, which has been previously polymerized to include such an active group, over the substrate surface with use of an appropriate solvent, and drying the surface.

As described above, the substrate for biological fluid treatment of the present invention can be produced such that: a recognition molecule is prepared by: (i) a step of translating a nucleic acid that encodes a recognition molecule having a selective binding property for at least one target substance, selected from a biorelated substance, a bacterium, a cell, a cell aggregation, a virus, or a virus-infected cell, into a polypeptide; (ii) a step of denaturing/solubilizing the recognition molecule polypeptide that has been insolubilized during the process of the above translation, with an aqueous solution containing guanidine hydrochloride or urea; and (iii) a step of, after the step (ii), renaturating the three-dimensional structure of the recognition molecule polypeptide by removing guanidine hydrochloride or urea: and the recognition molecule is then bound to the substrate surface. In addition, the substrate for biological fluid treatment of the present invention can also be produced by: (i) a step of introducing an active group into a surface of a water-insoluble substrate for forming a covalent bond with a recognition molecule having a selective binding property for at least one target substance, selected from a biorelated substance, a bacterium, a cell, a cell aggregation, a virus, or a virus-infected cell; and (ii) a step of covalently immobilizing the recognition molecule onto the substrate surface by contacting the water-insoluble substrate with an aqueous solution containing the recognition molecule at a temperature of 0°C to 50°C for 10 or more seconds. Such production methods of the substrate for biological fluid treatment of the present invention are also included in the scope of the present invention.

The substrate for biological fluid treatment according to the present invention that can be produced as mentioned above is able to constitute a device for biological fluid treatment by being loaded in a container having an inlet and an outlet. The "container having an inlet and an outlet" used herein is not specifically limited as long as it is capable of storing the substrate for biological fluid treatment. For example, a column or a tube can be used. The aforementioned device for biological fluid treatment is also included in the scope of the present invention.

The aforementioned device for biological fluid treatment can be used for the treatment against a disease associated with at least one target substance, selected from a biorelated substance, a bacterium, a cell, a cell aggregation, a virus, or a virus-infected cell. Examples of the disease associated with such target substance(s) can include, but not limited to: (1) acute renal failure, chronic renal failure, and the like; (2) fulminant hepatitis, acute hepatic failure, arteriosclerosia obliterans, myasthenia gravis, Guillain-Barre syndrome, multiple sclerosis, myeloma, drug poisoning, pemphigus, and the like; (3) septic shock, familial hyperlipemia, arteriosclerosia obliterans, focal glomerular sclerosis, dialytic amyloidosis, and the like; and (4) ulcerative colitis, chronic rheumatoid arthritis, multiple sclerosis, dermatosis, and immunological disease.

The target substance in an aqueous solution or blood can be adsorbed or removed by treating this aqueous solution or blood containing the target substance by flowing the aqueous solution or blood through the aforementioned device for biological fluid treatment. Such a treatment method of an aqueous solution and a treatment method of blood are also included in the present invention.

The present invention is hereafter described in greater detail with reference to the following examples.

Examples

As an example, a case is described in which a CD4 positive cell in human peripheral blood was used as the target substance, a single-chain antibody produced from an already known sequence of a monoclonal antibody Leu3a was used as the ligand site in recognition molecule, and streptavidin was used as the association domain.

Example 1: Production of Leu3a scFv-SA

(Production of Leu3a single-chain antibody gene)

Regarding the nucleic acid sequence of Leu3a being a mouse anti-human CD4 monoclonal antibody, Genbank Accession No. M97867.1 for the VH region and Genbank Accession No. M61046.1 for the VL region are open to the public. In order to form a single-chain antibody from these sequences, a sequence in which these sequences were linked by a nucleic acid that encodes a 15 amino acid sequence serving as a linker (SEQ ID NO: 1 of the sequence listing) in the order of VL-linker-VH, was designed and prepared by complete synthesis. At that time, an Nco I site was added to include an initiation codon right in front of the VL region, and a Not I site was added to the end of the VH region. These nucleic acids were subcloned into vector pUC57.

The complete synthesis and subcloning of the above nucleic acid sequence were done by the custom synthesis service provided by GenScript Corp., US. This nucleic acid sequence is shown in SEQ ID NO: 2 of the sequence listing with its amino acid three letter code, and the amino acid sequence alone is shown in SEQ ID NO: 3 of the sequence listing.

(Production of streptavidin core gene)

Regarding the nucleic acid sequence of streptavidin, Genbank Accession No. X03591 is open to the public. Of this sequence, the streptavidin core gene sequence corresponding to the amino acid residues 13-139 from the N terminal of the matured streptavidin protein was extracted, and codon optimization was carried out for better expression in *E coli.* without changing the amino acid sequence. Thereafter, a sequence in which a Xho I site was added to the 5' end, and a Sal I site, a His tag (SEQ ID NO: 4 of the sequence listing), a stop codon, and a Hind III site were added to the 3' end, was designed and prepared by complete synthesis. These nucleic acids were subcloned into vector pUC57. The complete synthesis, codon optimization for *E coli.* expression, and subcloning of the above nucleic acid sequence were done by the custom synthesis service provided by GenScript Corp., US. This nucleic acid sequence is shown in SEQ ID NO: 5 of the sequence listing with its amino acid three letter code, and the amino acid sequence alone is shown in SEQ ID NO: 6 of the sequence listing. In this application, a streptavidin protein derived from this sequence is abbreviated as SA.

(Production of spacer site)

The spacer comprises 13 amino acids of Not I site-derived 3 amino acids (AAA: amino acid one letter code), FLAG tag-derived 8 amino acids (SEQ ID NO: 7 of the sequence listing), Xho I site-derived 2 amino acids (SS: amino acid one letter code). The synthesis and subcloning thereof were done in the same manner. This nucleic acid sequence is shown in SEQ ID NO: 8 of the sequence listing with its amino acid three letter code, and the amino acid sequence alone is shown in SEQ ID NO: 9 of the sequence listing.

(Modification of *E. coli* expression vector)

A sequence between the restriction enzyme Not I site and the restriction enzyme Bpu1102I site was previously deleted from the multicloning site of plasmid pET24d(+) (NOVAGEN). The plasmid pET24d(+) was digested with the restriction enzyme Not I and the restriction enzyme Bpu1102I (TAKARA), and then was rendered blunt with Klenow Fragment (New England Biolabs) and dNTP mixture (TAKARA) according to the appended protocol. The resultant product was electrophoresed on 1% agarose gel, and stained with EtBr. Then, this DNA fragment was cut out, extracted, subjected to self-cyclization, and was then transformed into *E coli.* DH5 strain (TOYOBO), to thereby obtain the vector of interest. The DNA fragment extraction from the agarose gel was done with use of a kit of QIAGEN, and the ligation was done with use of ligation kit Ver.2 of TAKARA.

(Construction of expression vector)

The thus modified plasmid pET24d(+) was digested with the restriction enzyme Nco I and the restriction enzyme Hind III (TAKARA), and the vector's DNA fragment was purified in the same manner. The vector where the nucleic acid encoding Leu3a single-chain antibody was subcloned was digested with the restriction enzyme Nco I and the restriction enzyme Not I (TAKARA), and the nucleic acid fragment encoding Leu3a single-chain antibody was purified in the same manner. The vector where the nucleic acid encoding the spacer site was subcloned was digested with the restriction enzyme Not I and the restriction enzyme Xho I (TAKARA), and the nucleic acid fragment encoding the spacer site was purified in the same manner. The vector where the nucleic acid encoding the streptavidin core gene was subcloned was digested with the restriction enzyme Xho I and the restriction enzyme Hind III (TAKARA), and the nucleic acid fragment encoding the streptavidin core gene was purified in the same manner.

Thus purified respective DNA fragments were subjected to circular ligation with use of ligation kit Ver.2 of TAKARA, and were transformed into *E coli.* DH5 strain (TOYOBO) to obtain the expression vector expressing Leu3a scFv-SA protein.

The Leu3a scFv-SA protein which is produced with use of the above expression vector comprises Leu3a scFv (about 30 kD) as the "ligand site", 13 amino acids (about 2 kD) as the spacer site, and SA (about 16 kD) as the "association domain" having a self-association property. The molecular weight ratio of a polypeptide that constitutes the "ligand moiety" to a polypeptide that constitutes the "association domain" is 1.9:1 in this case.

Example 2: Expression, and recovery under denaturation condition with guanidine hydrochloride

The expression vector that had been constructed in Example 1 was transformed into *E coli.* BL21 (DE3) strain (NOVAGEN) according to the instruction manual. The strain was cultured in 2x YT medium (1.6% bacto triptone, 1% bacto yeast extract, and 0.5% NaCl) containing 34 $\mu$g/ml kanamycin and 1% glycerin at 37°C, and the culture was terminated when the absorbance OD at 600 nm reached 0.7 to 0.8. Glycerin was added at final concentration of 15%, and the mixture was stored as a glycerol stock at -80°C. 1 ml of the stored glycerol stock was mixed with 300 ml of 2x YT medium containing 34 $\mu$g/ml kanamycin and 1% glycerin, and was cultured at 37°C. IPTG (TAKARA) was added at final concentration of 1 mM when the absorbance OD at 600 nm reached 0.7 to 0.8. Incubation was then conducted for another 4 hours at 37°C. This *E coli.* suspension culture was transferred into a 500 ml centrifuge tube, and was subjected to centrifugal separation at 4,000 rpm for 15 minutes. Then, the bacterial pellet was collected. The bacterial pellet was suspended in 30 ml of sonication buffer (20 mM Tris-HCl, pH = 8.0, and 300 mM NaCl) and was sonicated in ice with an ultra sonic processor VP-60 (TAITEC) at an output of 5, a duty cycle of 50%, for 5 minutes, to break the bacteria. The resultant product was transferred into a 30 ml centrifuge tube, and was subjected to centrifugal separation at 10,000 rpm for 15 minutes. The supernatant was disposed to obtain an insoluble protein fraction in which inclusion proteins had been formed.

The fraction was suspended under breaking with 30 ml of a dissolving buffer (A) (50 mM Tris-HCl, pH = 8.0, 300 mM NaCl, and 6M Guanidine-HCl), and left to stand as it was at room temperature overnight. On the following day, centrifugal separation was carried out at 10,000 rpm for 15 minutes. The supernatant was collected to obtain crude Leu3a scFv-SA protein solubilized under denaturation with guanidine hydrochloride. This product was diluted with 70 ml of the same dissolving buffer and then contacted with 5 ml of TALON gel (Becton, Dickinson and Company: hereunder, expressed as BD) that had been previously equilibrated with the dissolving buffer (A) at room temperature for 2 hours, to effect adsorption of Leu3a scFv-SA protein. The resultant product was transferred into a disposable column (BD) for use as a TALON gel column, followed by washing with 25 ml of dissolving buffer (A). This column was diluted with 25 ml of a dilution buffer (B) (50 mM Tris-HCl, pH = 8.0, 300 mM NaCl, 6 M Guanidine-HCl, and 150 mM imidazole). 2-mercaptoethanol was added therein at a final concentration of 15 mM. The mixture was left to stand at room temperature for 1 hour, and filtrated through a 0.22 $\mu$m filter to obtain purified Leu3a scFv-SA protein solubilized under denaturation with guanidine hydrochloride. (Refolding and purification through dialysis with serial dilution of guanidine hydrochloride)

About 30 ml of the purified Leu3a scFv-SA protein which had been solubilized under denaturation with guanidine hydrochloride was dialyzed with 1 L of a dialysis external solution for the first step of serial dilution of guanidine hydrochloride (50 mM Tris-HCl, pH = 8.0, 300 mM NaCl, and 2 M Guanidine-HCl) at 4°C overnight using a Slide-A-Lyzer (molecular weight cutoff at 10 kD: PIERCE). Next, the resultant solution was further dialyzed with 1 L of a dialysis external solution for the second step of serial dilution of guanidine hydrochloride (50 mM Tris-HCl, pH = 8.0, 300 mM NaCl, 1 M Guanidine-HCl, 400 mM L-Arginine, and 375 $\mu$M oxidized glutathione) at 4°C for 24 hours. Then, in order to avoid precipitation after the renaturation of the three-dimensional structure of Leu3a scFv-SA protein, 5 ml of purified Leu3a scFv-SA protein was withdrawn during the above serial dilution dialysis, and was diluted with 25 ml of a dialysis external solution for the third step of serial dilution of guanidine hydrochloride salt (50 mM Tris-HCl, pH = 8.0, 300 mM NaCl, 0.5 M Guanidine-HCl, and 400 mM L-Arginine). This Leu3a scFv-SA protein solution in a total volume of 30 ml was again placed in a dialysis cassette of Slide-A-Lyzer (molecular weight cutoff at 10 kD: PIECE), and dialyzed with 1 L of the dialysis external solution for the third step of serial dilution of guanidine hydrochloride (50 mM Tris-HCl, pH = 8.0, 300 mM NaCl, 0.5 M Guanidine-HCl, and 400 mM L-Arginine) at 4°C overnight. Then, dialysis with 1 L of phosphate buffered saline (Dulbecco's PBS(-) solution: Nissui: hereunder, simply expressed as PBS(-)) at 4°C for 8 to 12 hours, was repeatedly conducted. Trace amounts of reprecipitated products generated during this process were removed through centrifugal separation and filtration using filters, to obtain refolded and purified Leu3a scFv-SA protein (hereunder, it is expressed as Leu3a scFv-SA-guanidine). The production amount of the Leu3a scFv-SA-guanidine purified in the above manner was 99 mg per liter of *E coli.* culture solution.

Example 3: Refolding and purification through dialysis with serial dilution of urea

About 30 ml of the Leu3a scFv-SA protein which had been purified and solubilized under denaturation with guanidine hydrochloride likewise of the former part of Example 2, was dialyzed with 1 L of a dialysis external solution for serial dilution of urea (50 mM Tris-HCl, pH = 8.0, 300 mM NaCl, and 4 M urea) at room temperature overnight using a Slide-A-Lyzer (molecular weight cutoff at 10 kD: PIERCE). Next, the resultant solution was further dialyzed with 1 L of a dialysis external solution for the second step of serial dilution of urea (50 mM Tris-HCl, pH = 8.0, 300 mM NaCl, 2 M urea, 400 mM L-Arginine, and 375 $\mu$M oxidized glutathione) at room temperature for 24 hours. Then, in order to avoid precipitation after the renaturation of the three-dimensional structure of Leu3a scFv-SA protein, 5 ml of purified Leu3a scFv-SA protein was withdrawn during the above serial dilution dialysis, and was diluted with 25 ml of a dialysis external solution for the third step of serial dilution of urea (50 mM Tris-HCl, pH = 8.0, 300 mM NaCl, 1M urea, and 400 mM L-Arginine). This Leu3a scFv-SA protein solution in a total volume of 30 ml was again placed in a dialysis cassette of Slide-A-Lyzer (molecular weight cutoff at 10 kD: PIECE), and dialyzed with 1 L of the dialysis external solution for the third step of serial dilution of urea (50 mM Tris-HCl, pH = 8.0, 300 mM NaCl, 1M urea, and 400 mM L-Arginine) at room temperature overnight. Then, dialysis with 1 L of phosphate buffered saline (Dulbecco's PBS(-) solution: Nissui: hereunder, simply expressed as PBS(-)) at 4°C for 8 to 12 hours, was repeatedly conducted. Trace amounts of reprecipitated products generated during this process were removed through centrifugal separation and filtration using filters, to obtain refolded and purified Leu3a scFv-SA protein (hereunder, it is expressed as Leu3a scFv-SA-Urea). The production amount of the Leu3a scFv-SA-Urea purified in the above manner was 102 mg per liter of *E coli.* culture solution.

Comparative Example 1:

For the purpose of comparison, a gene of a single-chain antibody which do not form a self-associated assembly was constructed by deleting the SA site of Leu3a scFv-SA of Example 1 and Example 2 in the following manner. Hereinunder, this gene is referred to as Leu3a scFv-Normal gene and a protein expressed therefrom is referred to as Leu3a scFv-Normal.

(Modification of spacer site-including C-terminal site)

An artificial gene in which Not I site, FLAG tag-derived 8 amino acids (SEQ ID NO: 7 of the sequence listing), and c-myc epitope tag-derived 10 amino acids (SEQ ID NO: 10 of the sequence listing) were included, and a His tag (SEQ ID NO: 4 of the sequence listing), a stop codon, a Xho I stop codon, a Sal I stop codon, and a Hind III site were introduced, was designed, synthesized, and subcloned in the same manner as that of Example 1.

This nucleic acid sequence is shown in SEQ ID NO: 11 of the sequence listing with its amino acid three letter code, and the amino acid sequence alone is shown in SEQ ID NO: 12 of the sequence listing.

(Construction of expression vector)

The modified plasmid pET24d(+) of Example 1 was digested with the restriction enzyme Nco I and the restriction enzyme Hind III (TAKARA), and the vector's DNA fragment was purified in the same manner. Likewise of Example 1, the vector where the nucleic acid encoding Leu3a single-chain antibody (Leu3a scFv-Normal) was subcloned was digested with the restriction enzyme Nco I and the restriction enzyme Not I (TAKARA), and the nucleic acid fragment encoding Leu3a single-chain antibody was purified in the same manner. The vector where the nucleic acid for modifying the aforementioned spacer site-including C-terminal site was subcloned was digested with the restriction enzyme Not I and the restriction enzyme Hind III (TAKARA), and the nucleic acid fragment for modifying the spacer site-including C-terminal site was purified in the same manner. Thus purified respective DNA fragments were subjected to circular ligation with use of ligation kit Ver.2 of TAKARA, and were transformed into *E coli.* DH5 strain (TOYOBO) to obtain the expression vector expressing Leu3a scFv-Normal protein.

The Leu3a scFv-Normal protein which is produced with use of the above expression vector comprises Leu3a scFv (about 30 kD) as the "ligand site" and 39 amino acids (about 5 kD) as the modified spacer site-containing C-terminal site, and lacks the association domain.

The expression vector produced in the above manner was subjected to (expression, and recovery under denaturation condition with guanidine hydrochloride) and (refolding and purification through dialysis with serial dilution of guanidine hydrochloride) likewise of Example 2. The production amount of the Leu3a scFv-Normal obtained in the above manner was 83 mg per liter of *E coli.* culture solution.

Example 4:

(GPC analysis)

Gel permeation chromatography (GPC analysis) was conducted in order to understand the state of assembly formation between Leu3a scFv-SA-guanidine, Leu3a scFv-SA-urea, and Leu3a scFv-Normal that had been produced in Example 2, Example 3, and Comparative Example 1. That is to say, respective samples were prepared in 100 $\mu$g/ml of PBS(-) solution, and 30 $\mu$l thereof was each applied into a gel permeation column (TOSOH, G4000PWXL). The identification was performed using PBS(-) as a running buffer at a flow rate of 0.8 ml/min, at the absorption of 225 nm. Gel Filtration standard (BIORAD) was used as the standard. The molecular weight of the assembly was calculated based on the peak top retention time. The results are shown in Table 1.

Table 1: GPC analysis between various recognition molecules

| Samples | Peak top retention time (min) | Apparent molecular weight of assembly (kD) |
|---|---|---|
| Leu3a scFv-SA-guanidine (Example 2) | 10.65 | 331 |
| Leu3a scFv-SA-urea (Example 3) | 10.95 | 225 |
| Leu3a scFv-Normal (Comparative Example 1) | 12.43 | 38 |

Leu3a scFv-SA-guanidine (Example 2) and Leu3a scFv-SA-Urea (Example 3) had greater molecular sizes, as compared to Leu3a scFv-Normal of Comparative Example 1, suggesting that their assemblies consisted of at least four or more molecules (four to eight molecules).

(SDS-PAGE analysis)

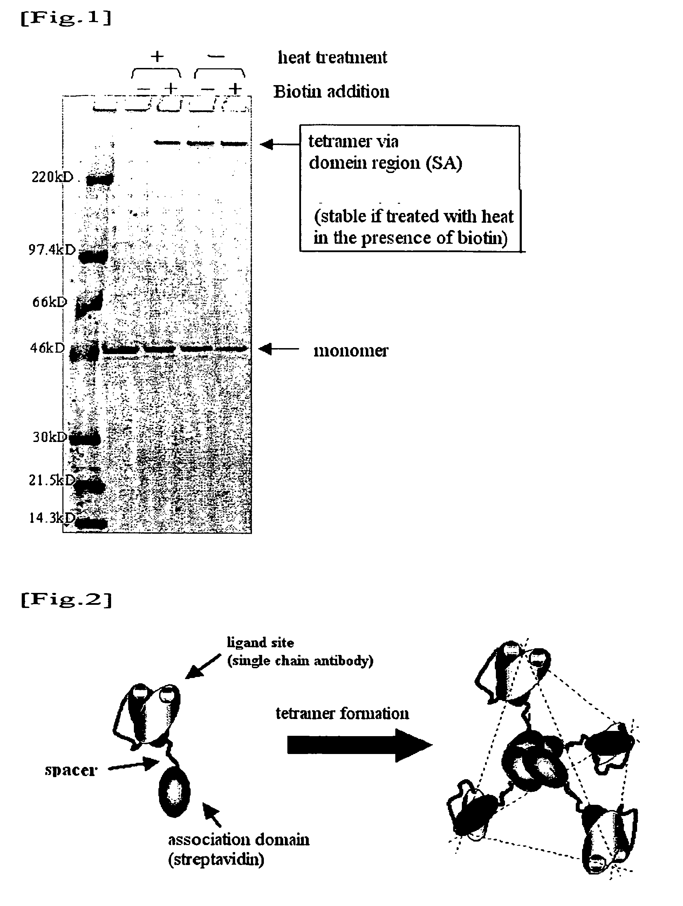

SDS-PAGE analysis of Leu3a scFv-SA-guanidine (Example 2) was conducted. That is to say, a predetermined amount (2 to 3 $\mu$g: 0.04 to 0.07 nmol) of Leu3a scFv-SA-guanidine was added with an excessive amount (5 nmol) of D-biotin(+) (SIGMA), and then was mixed with a SDS-PAGE sample buffer (containing 2-mercaptoethanol), followed by heat treatment at 99°C for 5 minutes. In the same manner as the above, a sample without biotin, and a sample which was not heat treated but left standing at room temperature were prepared. These were subjected to SDS-PAGE on 4% to 20% polyacrylamide gel (DaiichiKagaku), immobilization, and staining with CBB R-250 dye. The result is shown in Fig. 1.

It is known that streptavidin (SA) is prone to be associated into a tetramer, and biotin strengthens and retains the tetrameric assembly regardless of heat treatment (Proc. Natl. Acad. Sci. USA vol.92, pp. 3180-3184, 1995). SDS-PAGE analysis under the aforementioned condition enables to analyze whether or not tetramers are formed via SA portions.

As shown in the result of Fig. 1, some assemblies of Leu3a scFv-SA-guanidine formed strong tetramers under the presence of biotin regardless of heat treatment. Therefore, it can be understood that the tetrameric structure is particularly stable among self-associated assembly structures comprising four or more recognition molecules via their association domains (SA in this Example). In addition, even without the presence of biotin, when heat treatment was not applied, some assemblies formed tetrameric structures regardless of the presence of a strong surfactant SDS. Therefore, it can be understood that the tetrameric structure is stable even without the presence of biotin.

Leu3a scFv-SA-urea (Example 3) and Leu3a scFv-Normal (Comparative Example 1) were also analyzed in the same manner as the above. As a result, only bands of monomers were observed under the presence of a strong surfactant SDS.

Table 2 shows the state of assembly formation between respective recognition molecules indicated by the above results of GPC analysis and SDS-PAGE analysis.

Table 2: State of assembly formation between respective recognition molecules

| Recognition molecules | Number of recognition molecules per assembly | Number of recognition molecules in major assembly |
|---|---|---|
| Leu3a scFv-SA-guanidine (Example 2) | 4 to 8 molecules | 4 molecules |
| Leu3a scFv-SA-Urea (Example 3) | 4 to 8 molecules | 4 to 8 molecules |
| Leu3a scFv-Normal (Comparative Example 1) | 1 molecule | 1 molecule |

Example 5:

(Production of GMA grafted nonwoven fabric)

A polypropylene (hereunder, expressed as PP) nonwoven fabric P080HW-00F (TONEN TAPYRUS, average fiber diameter: 3.5 $\mu$m) was cut into 19 cm x 12 cm pieces, followed by degassing, nitrogen substitution, and sealing. Gamma rays of 200 kGy was irradiated thereto to obtain a PP nonwoven fabric having radicals on the substrate surface. Four sheets thereof were laminated. The four layered fabric was then transferred into a container in which oxygen was removed by means of sufficient nitrogen substitution, and immersed in 400 ml of methanol solution containing 10 v/v % glycidyl methacrylate (hereunder, referred to as GMA) at 40°C. After 25 minutes grafting reaction, the nonwoven fabric was taken out, washed with a large amount of methanol to remove unreacted GMA, and then dried under reduced pressure, to thereby obtain a GMA grafted PP nonwoven fabric. The calculation of the graft rate is defined by the following equation.

(Weight of nonwoven fabric after grafting - weight of nonwoven fabric before grafting) / weight of nonwoven fabric before grafting

The graft rate at this time was 89.1%. The GMA grafted nonwoven fabric has many glycidyl groups (epoxy group) as active groups, and is capable of covalently immobilizing a recognition substance through reaction with amino groups in proteins.

This GMA grafted nonwoven fabric was cut into circular pieces of 7 mm diameter. Four pieces thereof were immersed in ethanol, and mechanically squeezed to remove ethanol as much as possible. Then, the resultant product was immersed in 400 $\mu$l of each recognition molecule solution (16 $\mu$g of recognition molecules/400 $\mu$l) in which the recognition molecules were dissolved in PBS(-) containing 0.2% polyoxyethylene sorbitan monolaurate (hereunder, expressed as Tween20) (hereunder, expressed as PBS-T) at room temperature (about 23°C) for 19 hours under gentle shaking.

Then, the nonwoven fabric was transferred into 400 $\mu$l of PBS(-) solution containing 160 $\mu$M D-Biotin(+), to block the biotin-binding site in the SA portion.

The recognition molecules used herein were respectively Leu3a scFv-SA-guanidine, Leu3a scFv-SA-Urea, and Leu3a scFv-Normal produced in Example 2, Example 3, and Comparative Example 1. For another comparison, a fabric where commercially available anti-human CD4 monoclonal antibody (clone 4H5, Medical & Biological Laboratories) was immobilized was prepared, which is referred to as Comparative Example 2. In addition, another GMA grafted nonwoven fabric was subjected to the same procedures but was not immobilized any recognition molecule, which is referred to as Comparative Example 3. After the immobilization with various recognition molecules, washing operation under vigorous shaking with 20 ml of PBS-T solution was repeated three times, and additional washing operation with 20 ml of PBS(-) solution in the same manner was repeated twice, to wash out the recognition molecules bound to the surface by means of adsorption, so as to thereby obtain the desired substrates for biological fluid treatment in which respective recognition molecules were covalently immobilized on the substrate surface. By the above operation, the respective recognition molecules of Example 1, Comparative Example 1, and Comparative Example 2 were bound to the abovementioned activated nonwoven fabric surfaces via amino groups of the respective recognition molecules.

Four sheets of the abovementioned respective cell adsorbent, and PBS(-) solution, were loaded in a 1 ml container having an inlet and an outlet (Mobicol column: Funakoshi) to produce a device for biological fluid treatment.

(Evaluation of cell adsorptivity)

Immediately before the initiation of experiment, the PBS(-) solution was drained off by flowing air through the column. 5.0 ml of healthy human fresh blood containing ACD-A (KAWASUMI Laboratories) (blood:ACD-A = 6.7:1) was fed from the inlet of the cell adsorber at a flow rate of 1.0 ml/min with a syringe pump. After discarding the first five drops, the treated blood was collected from the outlet of the column.

The CD4 positive cell removal rate at that time was calculated based on the change in the number of leukocytes measured by Coulter counter, and the change in the proportion of CD4 positive cells in the recovered cells measured by flow cytometry with use of a flow cytometer (FACS Calibur: Becton, Dickinson and Company). The cells were stained by the following manner. 100 $\mu$l of the collected blood was added with 10 $\mu$l of anti-CD4-PE solution (clone SK3, Becton, Dickinson and Company), 10 $\mu$l of anti-CD8-FITC solution (clone RPA-T8, Becton, Dickinson and Company), and 10 $\mu$l of anti-CD45-PerCP solution (Becton, Dickinson and Company). The resultant mixture was allowed to be stained at room temperature for 30 minutes, and then was added with 1 ml of FACS Lysing solution (Becton, Dickinson and Company) to effect hemolysis. CD45 positive cells were gated as leukocytes, and the proportion of CD4 positive cells therein was assayed. In addition, in order to evaluate the specificity to the target substance, the recovery rate of CD8 positive cells, which have quite similar properties to those of CD4 positive cells but do not have CD4 molecules on the surface, was shown together. These results are shown in Table 3 by mean values $\pm$ SD from three experiments for Example 2, Example 3, and Comparative Example 1, and by values from one-off experiment for Comparative Example 2 and Comparative Example 3.

Table 3: CD4 positive cell removal rate and CD8 cell recovery rate in respective recognition molecules

| Recognition molecules immobilized on the surface | CD4 positive cell removal rate (%) | CD8 positive cell recovery rate (%) |
|---|---|---|
| Leu3a scFv-SA-guanidine (Example 2) | 91.4$\pm$2.5 | 76.1$\pm$3.4 |
| Leu3a scFv-SA-Urea (Example 3) | 62.8$\pm$4.1 | 77.9$\pm$6.1 |
| Leu3a scFv-Normal (Comparative Example 1) | 29.6$\pm$4.4 | 78.9$\pm$1.1 |
| 4H5 antibody (Comparative Example 2) | 32.7 | 72.2 |
| Without recognition molecule (Comparative Example 3) | 21.3 | 81.6 |

These results show that covalent immobilization of recognition molecules onto the substrate surface under the immobilization condition of Example 5 is remarkably effective in the adsorption/removal of the target substance, CD4 positive cells, when "at least four or more recognition molecules form an assembly structure through self-association of their association domains" referred to in the present invention, without causing nonspecific adsorption/removal of CD8 positive cells. This is superior to the commercially available anti-human CD4 antibody in the adsorption/removal effect when immobilized under the same condition. In addition, it is understood that the effect is particularly notable when using Leu3a scFv-SA-guanidine as the recognition molecule (Example 2), molecules of which are mainly associated into a tetrameric assembly (from Example 4).

Example 6:

(Introduction of biotin PEO-amine into the surface of GMA grafted nonwoven fabric)

A GMA grafted nonwoven fabric was produced in the same manner as that of Example 5. The GMA graft rate at this time was 81.4%. This GMA grafted nonwoven fabric was cut into circular pieces of 7 mm diameter. Four pieces thereof were immersed in 400 $\mu$l of methanol solution containing 10 mM ($10^{-5}$ mol/ml) biotin PEO-amine (PIECE), and allowed to react at 37°C for 48 hours. The resultant product was washed twice with methanol, twice with PBS-T, and twice with distilled water, and then was dried under reduced pressure, to thereby produce a substrate to the surface of which was bound with biotin via PEO chains and amino groups (hereunder, expressed as biotin-aminated GMA-g-nonwoven fabric).

(Introduction of biotin hydrazine into the surface of GMA grafted nonwoven fabric)

A GMA grafted nonwoven fabric was produced in the same manner as that of Example 5. The GMA graft rate at this time was 81.4%. This GMA grafted nonwoven fabric was cut into circular pieces of 7 mm diameter. Four pieces thereof were immersed in 400 $\mu$l of methanol solution containing 1 mM ($10^{-6}$ mol/ml) biotin hydrazine (SIGMA), and allowed to react at 37°C for 48 hours. The resultant product was washed twice with methanol, twice with PBS-T, and twice with distilled water, and then was dried under reduced pressure, to thereby produce a substrate to the surface of which was bound with biotin via hydrazide groups (hereunder, expressed as biotin-hydrazinated GMA-g-nonwoven fabric).

The thus produced GMA grafted nonwoven fabric, biotin-aminated GMA-g-nonwoven fabric, and biotin-hydrazinated GMA-g-nonwoven fabric were immersed in ethanol, and mechanically squeezed to remove ethanol as much as possible. Then, the resultant products were immersed in 400 $\mu$l of PBS-T solution containing 16 $\mu$g of Leu3a scFv-SA-guanidine as the recognition molecule (16 $\mu$g/400 $\mu$l) at room temperature (about 23°C) for 19 hours under gentle shaking. Then, the nonwoven fabrics were transferred into 400 $\mu$l of PBS(-) solution containing 160 $\mu$M D-Biotin(+), to block the biotin-binding site in the SA portion.

By the above procedure, a substrate in which Leu3a scFv-SA-guanidine as recognition molecule had been covalently introduced into the surface (GMA grafted nonwoven fabric), and substrates in which Leu3a scFv-SA-guanidine had been introduced mainly by means of noncovalent biotin-streptavidin (biotin-SA) bonds via biotin on the surface (biotin-aminated GMA-g-nonwoven fabric, and biotin-hydrazinated GMA-g-nonwoven fabric), were obtained.

Four sheets of the abovementioned respective cell adsorbent, and PBS(-) solution, were loaded in a 1 ml container having an inlet and an outlet (Mobicol column: Funakoshi) to produce a device for biological fluid treatment.

(Evaluation of cell adsorptivity)

Evaluation of cell adsorptivity was performed in the same manner as that of Example 5. The results are shown in Table 4 with CD4 positive cell (target substance) removal rate.

Table 4: CD4 positive cell removal rate in respective substrates

| Substrates bound with Leu3a scFv-SA-guanidine | CD4 positive cell removal rate (%) |
|---|---|
| GMA grafted nonwoven fabric (mainly by covalent bond) | 72.8 |
| Biotin-aminated GMA-g-nonwoven fabric (mainly by noncovalent biotin-SA bond) | 55.7 |
| Biotin-hydrazinated GMA-g-nonwoven fabric (mainly by noncovalent biotin-SA bond) | 19.0 |

These results surprisingly show that, for the immobilization of Leu3a scFv-SA-guanidine as recognition molecule produced in Example 2 onto the substrate surface, easily achievable covalent immobilization of the recognition molecule onto the substrate surface is more effective in the selective removal of the target substance, rather than directional immobilization, as exemplified in Non-patent Document 1, in which biotin has been effectively introduced in the substrate surface mainly by means of biotin-SA bond using SA, an association domain in the recognition molecule. It is exemplified in Example 4, that Leu3a scFv-SA-guanidine was firmly bound to biotin to form a stable tetramer in an associated state consisting of four or more molecules. For this reason, it is understood that the substrate for biological fluid treatment made by such a covalent immobilization onto the substrate surface is superior, when the recognition molecule is a linear molecule comprising a ligand moiety which selectively binds to the target substance, and an association domain, and at least four or more recognition molecules form an assembly structure through self association of their association domains.

In addition, biotin-hydrazinated GMA-g-nonwoven fabric showed an inferior result in the performance of selective removal of CD4 positive cells which is the target substance. The reason can be considered to be the variation in the charge state and/or degree of hydrophilicity/hydrophobicity of the surface of nonwoven fabric, between the biotin-hydrazinated GMA-g-nonwoven fabric produced using biotin hydrazine and the biotin-aminated GMA-g-nonwoven fabric. As a result, physical adsorption as well as biotin-SA bond might have caused additional change in the orientation of the recognition molecules, in the immobilization of recognition molecules onto the substrate surface. In this way, the binding activity of the recognition molecule may not be retained sometimes depending on the method for introducing biotin into the substrate surface in cases of immobilization achieved mainly by means of noncovalent biotin-SA bond.

Example 7:

A GMA grafted nonwoven fabric was produced in the same manner as that of Example 5. The GMA graft rate at this time was 81.4%. This GMA grafted nonwoven fabric was cut into circular pieces of 7 mm diameter. Four pieces thereof were immersed in ethanol, and mechanically squeezed to remove ethanol as much as possible. Then, the resultant product was immersed in 400 $\mu$l of PBS-T solution containing 16 $\mu$g of Leu3a scFv-SA-guanidine as the recognition molecule (16 $\mu$g/400 $\mu$l) at room temperature (about 23°C) for 19 hours under gentle shaking. Then, two types of substrates for biological fluid treatment were produced from the above nonwoven fabric: one was a case in which the fabric was transferred into 400 $\mu$l of PBS(-) solution containing 160 $\mu$M D-Biotin(+), to block the biotin-binding site in the SA portion (hereunder, expressed as with biotin block); and the other was a case in which the fabric was transferred into 400 $\mu$l of normal PBS(-) solution without D-Biotin(+), followed by the same procedures (hereunder, expressed as without biotin block).

Four sheets of the abovementioned respective cell adsorbent, and PBS(-) solution, were loaded in a 1 ml container having an inlet and an outlet (Mobicol column: Funakoshi) to produce a device for biological fluid treatment.

(Evaluation of cell adsorptivity)

Evaluation of cell adsorptivity was performed in the same manner as that of Example 5. The results are shown in Table 5 by mean values $\pm$ SD from three experiments.

Table 5: CD4 positive cell removal rate and CD8 cell recovery rate with and without biotin block

| With or without biotin block after immobilization of recognition molecule | CD4 positive cell removal rate (%) | CD8 positive cell recovery rate (%) |
|---|---|---|
| With biotin block | 75.3$\pm$2.5 | 82.4$\pm$2.0 |
| Without biotin block | 74.5$\pm$4.1 | 82.0$\pm$1.7 |

These results show that totally equivalent performance in removal of the target substance was demonstrated between with or without the binding of biotin to the SA biotin-binding site serving as the association domain, once the recognition molecules have been covalently immobilized onto the substrate surface. This shows that biotin is totally unnecessary for the performance in removal of the target substance, with the substrate for biological fluid treatment of the present invention, although SA is used for the domain site.

Example 8:

GMA grafted nonwoven fabrics were produced in the same manner as that of Example 5. In this case, however, nonwoven fabrics having different graft rates were prepared with a variation of the GMA concentration in methanol solution within a range of 10% and 20% (v/v %). The GMA graft rates at this time were 53.8%, 81.4%, 121.6%, and 186.4% in the order of lowest to highest. These GMA grafted nonwoven fabrics were cut into circular pieces of 7 mm diameter. Four pieces thereof were immersed in ethanol, and mechanically squeezed to remove ethanol as much as possible. Then, the resultant products were immersed in 400 $\mu$l of PBS-T solution containing 16 $\mu$g of Leu3a scFv-SA-guanidine as the recognition molecule (16 $\mu$g/400 $\mu$l) at room temperature (about 23°C) for 19 hours under gentle shaking. Then, the nonwoven fabrics were transferred into 400 $\mu$l of PBS(-) solution containing 160 $\mu$M D-Biotin(+), to block the biotin-binding site in the SA portion, so as to thereby produce four types of substrates for biological fluid treatment having different graft rates.

Four sheets of the abovementioned respective cell adsorbent, and PBS(-) solution, were loaded in a 1 ml container having an inlet and an outlet (Mobicol column: Funakoshi) to produce a device for biological fluid treatment.

(Evaluation of cell adsorptivity)

Evaluation of cell adsorptivity was performed in the same manner as that of Example 5. The results are shown in Table 6 by mean values $\pm$ SD from three experiments.

Table 6: CD4 positive cell removal rate and CD8 cell recovery rate in various GMA graft rates

| GMA graft rates of substrate surface (%) | CD4 positive cell removal rate (%) | CD8 positive cell recovery rate (%) |
|---|---|---|
| 53.8 | 86.3$\pm$1.6 | 72.6$\pm$1.1 |
| 81.4 | 84.8$\pm$2.9 | 74.7$\pm$3.1 |
| 121.6 | 86.1$\pm$4.4 | 76.0$\pm$4.2 |

(continued)

| GMA graft rates of substrate surface (%) | CD4 positive cell removal rate (%) | CD8 positive cell recovery rate (%) |
|---|---|---|
| 186.4 | 82.2±6.6 | 77.5±3.5 |

These results show that the performance in removal of the target substance is not affected at all, as long as a certain amount of the active group (GMA epoxy group) is present in the substrate surface for the covalent immobilization of the recognition molecule onto the substrate surface, and such a material can be suitably used for the substrate for biological fluid treatment of the present invention.

Hereunder is another example of the present invention in which a CD19 positive cell in human peripheral blood was used as the target substance, a single-chain antibody produced from an already known sequence of a monoclonal antibody 4G7 was used as the ligand site in recognition molecule, and streptavidin was used as the association domain.

Example 9: Production of 4G7 ScFv-SA

(Production of 4G7 single-chain antibody gene)

Regarding the nucleic acid sequence of 4G7 which is a mouse anti-human CD19 monoclonal antibody, Genbank Accession No. AJ555622 for the VH region and Genbank Accession No. AJ555479 for the VL region are open to the public. In order to form a single-chain antibody from these sequences, a sequence in which these sequences were ligated by a nucleic acid that encodes a 15 amino acid sequence serving as a linker (SEQ ID NO: 1 of the sequence listing) in the order of VL-linker-VH, was designed and prepared by complete synthesis. At that time, an Nco I site was added to include an initiation codon right in front of the VL region, and a Not I site was added at the end of the VH region. These nucleic acids were subcloned into vector pUC57.

The complete synthesis and subcloning of the above nucleic acid sequence were done by the custom synthesis service provided by GenScript Corp., US. This nucleic acid sequence is shown in SEQ ID NO: 13 of the sequence listing with its amino acid three letter code, and the amino acid sequence alone is shown in SEQ ID NO: 14 of the sequence listing.

Production of the streptavidin core gene, production of the spacer site, and modification of the *E. coli* expression vector were carried out in the same manners as those of Example 1, and construction of the expression vector was carried out in the following manner.

The modified plasmid pET24d(+) of Example 1 was digested with the restriction enzyme Nco I and the restriction enzyme Hind III (TAKARA), and the DNA fragment of the vector was purified in the same manner. The vector where the nucleic acid encoding 4G7 single-chain antibody was subcloned was digested with the restriction enzyme Nco I and the restriction enzyme Not I (TAKARA), and the nucleic acid fragment encoding 4G7 single-chain antibody was purified in the same manner. The vector where the nucleic acid encoding the spacer site was subcloned was digested with the restriction enzyme Not I and the restriction enzyme Xho I (TAKARA), and the nucleic acid fragment encoding the spacer site was purified in the same manner. The vector where the nucleic acid encoding the streptavidin core gene was subcloned was digested with the restriction enzyme Xho I and the restriction enzyme Hind III (TAKARA), and the nucleic acid fragment encoding the streptavidin core gene was purified in the same manner.

The thus purified respective DNA fragments were subjected to circular ligation with use of ligation kit Ver.2 of TAKARA, and were transformed into *E coli.* DH5 strain (TOYOBO) to obtain the expression vector expressing 4G7 scFv-SA protein.

The 4G7 scFv-SA protein produced with use of the above expression vector comprises 4G7 scFv (about 29 kD) as the "ligand site", 13 amino acids (about 2 kD) as the spacer site, and SA (about 16 kD) as the "association domain" having a self-association property. The molecular weight ratio of a polypeptide that constitutes the "ligand moiety" to a polypeptide that constitutes the "association domain" is 1.8:1 in this case.

(Expression, and recovery under denaturation condition with guanidine hydrochloride)

The expression vector that had been constructed in the above manner was subjected to expression, denaturation/solubilization, and refolding/purification through dialysis with serial dilution of guanidine hydrochloride likewise of Example 2. In this manner, refolded and purified 4G7 scFv-SA protein (hereunder, it is simply expressed as 4G7 scFv-SA) was obtained. The production amount of the 4G7 scFv-SA purified in the above manner was 148 mg per liter of *E coli.* culture solution.

A GMA grafted nonwoven fabric was produced in the same manner as that of Example 5. The GMA graft rate at this time was 89.1%. This GMA grafted nonwoven fabric was cut into circular pieces of 7 mm diameter. Four pieces thereof were immersed in ethanol, and mechanically squeezed to remove ethanol as much as possible. Then, the resultant product was immersed in 400 $\mu$l of PBS-T solution containing 80 $\mu$g of 4G7 scFv-SA as the recognition molecule (80 $\mu$g/400 $\mu$l) at room temperature (about 23°C) for 19 hours under gentle shaking. Then, two types of substrates for biological fluid treatment were produced from the above nonwoven fabric: one was a case in which the fabric was transferred into 400 $\mu$l of PBS(-) solution containing 160 $\mu$M D-Biotin(+), to block the biotin-binding site in the SA portion (hereunder, referred to as "with biotin block"); and the other was a case in which the fabric was transferred into 400 $\mu$l of normal PBS(-) solution without D-Biotin(+), followed by the same procedures (hereunder, referred to as "without biotin block"). In addition, another GMA grafted nonwoven fabric was subjected to the same procedures but was not immobilized with any recognition molecule, which is referred to as Comparative Example 4.

Four sheets of the abovementioned respective cell adsorbent, and PBS(-) solution, were loaded in a 1 ml container having an inlet and an outlet (Mobicol column: Funakoshi) to produce a device for biological fluid treatment.

(Evaluation of cell adsorptivity)

Immediately before the initiation of experiment, the PBS(-) solution was drained off by flowing air through the column. 5.0 ml of healthy human fresh blood containing ACD-A (KAWASUMI Laboratories) (blood:ACD-A = 6.7:1) was fed from the inlet of the cell adsorber at a flow rate of 1.0 ml/min with a syringe pump. After discarding the first five drops, the treated blood was collected from the outlet of the column.

The CD19 positive cell removal rate at that time was calculated based on the change in the number of leukocytes measured by Coulter counter, and the change in the proportion of CD19 positive cells in the recovered cells measured by flow cytometry with use of a flow cytometer (FACS Calibur: Becton, Dickinson and Company). The cells were stained by the following manner. 100 $\mu$l of the collected blood was added with 10 $\mu$l of Cyto-Stat/Coulter Clone solution (mixture of anti-CD19-FITC and anti-CD2-RD1, Becton Coulter), and 10 $\mu$l of anti-CD45-PerCP solution (Becton, Dickinson and Company). The resultant mixture was allowed to be stained at room temperature for 30 minutes, and then was added with 1 ml of FACS Lysing solution (Becton, Dickinson and Company) to effect hemolysis. CD45 positive cells were gated as leukocytes, and the proportion of CD 19 positive cells therein was assayed. In addition, in order to evaluate the specificity to the target substance, the recovery rate of CD2 positive cells, which have quite similar properties to those of CD 19 positive cells but do not have CD 19 molecules on the surface, was shown together. These results are shown in Table 7 by mean values $\pm$ SD from three experiments, except for Comparative Example 4 without recognition molecule the results of which were shown by values from one-off experiment.

Table 7: CD19 positive cell removal rate and CD2 cell recovery rate with 4G7 scFv-SA as recognition molecule

| Immobilized recognition molecules (biotin block) | CD19 positive cell removal rate (%) | CD2 positive cell recovery rate (%) |
|---|---|---|
| 4G7 scFv-SA immobilization (with biotin block) | 67.1±2.0 | 78.7±2.4 |
| 4G7 scFv-SA immobilization (without biotin block) | 68.7±1.9 | 76.4±1.3 |
| Without recognition molecule (Comparative Example 4) | 28.4 | 86.4 |

These results show that the substrate for biological fluid treatment of the present invention is capable of selective removal of different target substances by changing the ligand site of the recognition molecule which selectively binds to the target substance. In addition, likewise of Example 7, it is shown that biotin is totally unnecessary for the performance in removal of the target substance, although SA is used for the domain site.

Example 10:

A GMA grafted nonwoven fabric was produced in the same manner as that of Example 5. The GMA graft rate at this time was 81.4%. This GMA grafted nonwoven fabric was cut into pieces of 10 cm x 10 cm. Ten pieces thereof were immersed in ethanol, and mechanically squeezed to remove ethanol as much as possible. Then, the resultant product was immersed in 240 ml of PBS-T solution having Leu3a scFv-SA-guanidine dissolved therein (9.6 mg of recognition molecules/240 ml) at room temperature (about 23°C) for 19 hours under gentle shaking.

After the immobilization with recognition molecules, washing operation under vigorous shaking with 4 L of PBS-T solution was repeated three times, and additional washing operation with 4 L of PBS(-) solution in the same manner was repeated twice, to wash out the recognition molecules bound to the surface by means of adsorption, so as to thereby obtain the desired substrate for biological fluid treatment in which respective recognition molecule was covalently immobilized on the substrate surface.

Ten sheets of the abovementioned substrate for biological fluid treatment, PBS(-) solution, and ascorbic acid were loaded in a container having an inlet and an outlet, followed by sterilization with 25 kGy radiation. By so doing, an extracorporeal blood circulating device for the treatment of autoimmune disease targeting at CD4 positive cells was produced.

Reference Example:

In order to clarify the difference from a conventional technique, the production method of the substrate for biological fluid treatment and its experimental results described in Non-patent Document 1 were analyzed in detail and prophetically compared with the present invention. The physical properties of magnetic beads used in Non-patent Document 1 are described in the data sheet of Dynal's (Norway) Dynabeads M-280, sheep anti-mouse IgG (Prod. Nos. 112.01 and 112.02, Rev. No. 004), and it is possible to calculate the amount and the surface area of magnetic beads used in the experiment. In Non-patent Document 1, upon preparation of the substrate for biotin-mediated immobilization of the recognition molecule, 400 $\mu$l of solution was used for chemically binding biotin to amino groups of sheep anti-mouse antibodies on the surface of magnetic beads, which corresponds to 2.4 x 10$^8$ beads. From the formula of surface area of sphere ($4\pi r^2$) of beads having a diameter of 2.8 $\mu$m, 2.46 x 10$^{-7}$ cm$^2$/beads is obtained and thus the total surface area of magnetic beads used in the reaction becomes about 59.1 cm$^2$. These beads were reacted with N-hydroxysuccinimidobiotin, washed, and contacted with a solution containing 0.1 mg/ml single-chain antibody-streptavidin fusion protein which is a recognition molecule to effect immobilization of the recognition molecule via biotin on the surface of the magnetic beads. The amount of the recognition molecule used herein can be estimated as follows. Considering that the maximum is 1 ml in view of the volume of the microcentrifuge tube used in the reaction, and the required minimum is about 100 $\mu$l for evenly contacting 400 ul of beads with the solution of the recognition molecule, the amount of the recognition molecule used for biotin-mediated immobilization onto the surface of the magnetic beads becomes about 10 to 100 $\mu$g. Accordingly, the amount of the recognition molecule used for biotin-mediated immobilization on the surface per unit area can be calculated to be about 0.17 to 1.7 $\mu$g/cm$^2$.

In Non-patent Document 1, thereafter, the magnetic beads were washed and then resuspended at 6 x 10$^5$ beads/ul (10 $\mu$g/ul). 50 ul thereof (one eighth of the initial amount used for biotination and biotin mediated immobilization reaction of the recognition molecule, that is 3 x 10$^7$ beads, about 7.4 cm$^2$ as the surface area to be bound with the target substance to be removed) was contacted with 1 ml of a biological fluid containing target bacterial spores at a concentration of 5 x 10$^4$ CFU/ml (the absolute amount of spores was 5 x 10$^4$ CFU) at room temperature or at 4°C for 1 hour. This shows that such a procedure was capable of adsorbing and separating 90% or more of the target bacterial spores onto the surface of the magnetic beads.

This result is based on the case where phosphate physiological saline (PBS) containing 0.1% bovine serum albumin (BSA) was mixed with the target bacterial spores (*Bacillus cereus* spores) for use as the biological fluid, and any other impurities were contained therein. Further, in Non-patent Document 1, a mixture of milk and the target bacterial spores was subjected to the same examination; in which case, the adsorption and removal performance was only 37%. It was described that the case containing impurities such as milk resulted in remarkable reduction in the performance.

On the other hand, with regard to the substrate for biological fluid treatment described in this application, human whole blood added with an anticoagulant alone was used as described in a plurality of Examples. The target was not only those merely containing liquid protein, salt, or lipid, but also those containing various impurities such as erythrocytes and platelets, which are granular hemocytes more abundant than leukocytes serving as the target in Examples, and other lymphocytes having quite similar physical nature to leukocytes of the target substance. As the standard condition of Examples in this application, 16 $\mu$g of the recognition molecule was immobilized onto a lamination of four sheets of nonwoven fabrics cut into circular pieces of 7 mm diameter (140 to 190 cm$^2$ as the surface area to be bound with the target substance). Accordingly, the amount of the recognition molecule used for covalent immobilization on the surface per unit area can be calculated to be about 0.084 to 0.114 $\mu$g/cm$^2$. This shows that the amount of the recognition molecule supplied for immobilization reaction onto the surface, or the amount of the recognition molecule used per unit area, was not largely different or only about one tenth order, as compared to Non-patent Document 1.

The calculation method of theoretical effective surface area of the surface of the nonwoven fabric of the present application provides the surface area under an assumption that a column-shaped fiber constitutes the nonwoven fabric, which is calculated by the following equation.

$$\text{Effective surface area } (m^2/g) = 4 \text{ /relative density of the material of nonwoven}$$

$$\text{fabric } (d) \times \text{ average fiber diameter of nonwoven fabric } (\mu m)$$

Incidentally, the number of CD4 positive cells, one of the target substances shown in Examples, in blood can be estimated to be about $1 \times 10^6$ cells/ml, because 960 cells/ul can be obtained from an assumption that the number of leukocytes in peripheral blood is 6,000 cells/ul ($6 \times 10^6$ cells/ml), the proportion of lymphocytes in peripheral blood is 40%, and the proportion of CD4 positive cells therein is 40%. These fabrics were packed into a column having an inlet and an outlet, and 5 ml of human whole blood, which was a biological fluid containing not only the removal target substance but also large amounts of impurities, added with an anticoagulant alone, was fed therein at a flow rate of 1 ml/min (the contact time with the substrate was 5 minutes in total). Regardless of such a short contact time, it was possible to remove 60% to 90% or more of the removal target substance, and to recover 70% or more of other lymphocytes having quite similar physical nature to the target of removal. This shows that, as compared to the Non-patent Document 1 mentioned above, the substrate of the present application is capable of treating centuple amount of removal target substance (as compared to Non-patent Document 1 with the amount of spores at $5 \times 10^4$ CFU/ml x 1 ml, while the present invention with the amount of cells at $1 \times 10^6$ cells/ml x 5 ml), in one twelfth of the treatment time (5 minutes as compared to 60 minutes), regardless of the equivalent or one tenth order of the amount of the recognition molecule used for the immobilization reaction onto the surface. The substrate described in the present application is shown to be superior to the conventional technique in terms of the activity and performance.

It is implied that one of the causative factors of such a result might be a low efficiency due to steric hindrance involved in the binding between biotin introduced into the substrate surface and the streptavidin's biotin-binding site located in the center of the single-chain antibody-streptavidin fusion protein serving as the recognition molecule that forms a tetramer or higher order polymer, as described in Non-patent Document 1. In addition, a tetrahedron structure having ligand moieties at apical positions is considered to be stable for the recognition molecules to form a tetramer with streptavidin as association domain. Fig. 2 shows a schematic diagram of an assembly structure (tetramer) of recognition molecules. That is, it is highly possible that the tetrahedron structure of the assembly of recognition molecules is distorted for the biotin-mediated immobilization of the recognition molecule onto the substrate surface; as a result of which, high proportion of the ligand site is considered to be immobilized inefficiently in a form incapable of recognizing the target molecule. Alternatively, it is considered that only a part of the recognition molecule supplied for immobilization was actually immobilized via biotin in the immobilization method of Non-patent Document 1.

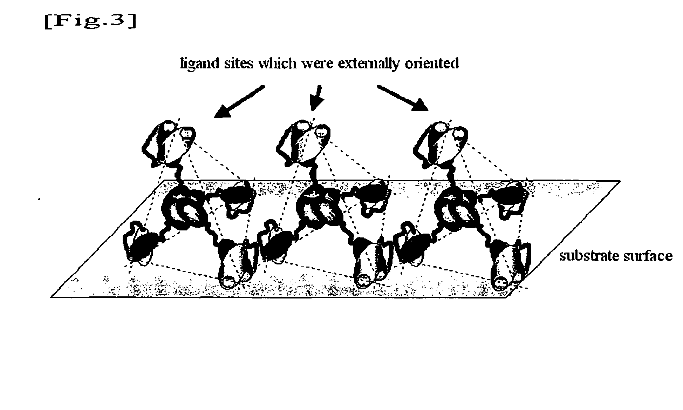

On the other hand, immobilization onto the surface while retaining the tetrahedron structure is considered to be possible if the recognition molecules are covalently immobilized by the method described in the present application. In this case, at least one apical position among four apical positions for coordinating the ligand sites are(is) immobilized in a form externally oriented with respect to the substrate surface. In addition, since large amounts of active groups for forming covalent bonds are present on the surface, greater amounts of recognition molecules can be immobilized. This is considered to have resulted in a totally unexpected effect, such that quite large amounts of recognition molecules were directionally immobilized while the ligand sites of at least one apical position were being externally oriented. This shows that an outstanding effect can be exerted as the substrate for biological fluid treatment even if three apical positions out of four apical positions of the ligand sites were deactivated due to the covalent bond with the substrate surface. It is quite difficult for those skilled in the art to expect this effect. Fig. 3 shows a schematic diagram of an immobilized state of recognition molecules and an orientated state of ligand sites on the substrate surface of the present invention.

In addition, it is understood that the treated amount of the removal target substance is substantially the same on the basis of calculation assuming that the surface area capable of adsorbing the removal target substance is equally 100 cm$^2$ (Non-patent Document 1 showed about $6.75 \times 10^5$ CFU/100 cm$^2$, while Example of the present invention showed 5.3 to $7.1 \times 10^5$ cells/100 cm$^2$), whereas the present invention has achieved the separation in one twelfth of the treatment time (5 minutes as compared to 60 minutes). Further, in consideration of the existence of large amounts of impurities, it can be understood that the present invention is outstandingly superior to Non-patent Document 1 in terms of the performance, on the basis of comparison assuming that the surface area capable of adsorbing the removal target substance is equal.

Further, the diameter of *Bacillus* spore used in Non-patent Document 1 is about 0.9 to 1.7 $\mu$m according to a reference document (Journal of Applied Microbiology, February 2007, 102, pp. 303-312), which is smaller than 10 to 20 $\mu$m which is the diameter of leukocytes serving as the target of Examples in the present application. Therefore, a greater number of the target spores is supposed to be adsorbed onto the substrate of Non-patent Document 1 than the number of leukocytes of Examples in this application per unit area. That is to say, these differences are not due to physical insufficiency of the surface area of substrate.

Industrial Applicability

Not only in cytapheresis, but also in general medical techniques for separating and treating blood cell components, it has been expected to improve the safety and therapeutic effect as well as reducing side effects by selectively and specifically capturing pathogenic cells which are the target substance being a theoretically presumed etiology, through biological interaction such as immune reaction. However, no practically applicable technique which specifically captures cells which are the target substance in blood, in particular directly from the whole blood, has been established. The present invention is able to provide a practically applicable substrate for blood treatment, device, and separation method for directly and specifically capturing cells which are the target substance from the whole blood, which thus contributes to the improvement of medical quality and efficiency in the era of Evidence Based Medicine (EBM) because the target substance of interest can be solely treated.

In addition, the present invention is also able to provide an inexpensive and industrially applicable substrate for biological fluid treatment, device, and separation method, for the separation of the target substance from a non-blood biological fluid, without a need of complicated and expensive manipulations of previously immobilizing biotin onto the substrate surface and then non-covalently immobilizing the target molecule through the affinity of biotin-streptavidin, which can thus contribute to broader use of the separation and detection method over a wide range including the fields of food, inspection, and environment.

Brief Description of the Drawings

Fig. 1 shows the result of SDS-PAGE analysis with Leu3a scFv-SA-guanidine (Example 2).
Fig. 2 shows a schematic diagram of an assembly structure (tetramer) of recognition molecules.
Fig. 3 shows a schematic diagram of an immobilized state of recognition molecules and an orientated state of ligand sites on the substrate surface of the present invention.

SEQUENCE LISTING

<110> Asahi Kasei Kabushiki Kaisha
<120> Base material for treating biological fluids
<130> F107040-WO
<160> 14
<210> 1
<211> 15
<212> PRT
<213> Artificial Sequence
<220>
<223> flexible linker. Designed peptide based on length, flexibility and
hydrophilicity to act as a linker between VL and VH of single chain Fv mo
lecule
<400> 1

Gly Gly Gly Gly Ser Gly Gly Gly Gly Ser Gly Gly Gly Gly Ser
1               5                   10                  15

<210> 2
<211> 748
<212> DNA
<213> mouse and artificial sequence
<220>
<223> Single chain Fv. anti-Leu3a VL and VH fragment was connected with f
lexible linker. Modified with restriction enzyme site.
<400> 2

```
cc atg gcc gac att gtg ctg acc caa tct cca gct tct ttg gct gtg      47
   Met Ala Asp Ile Val Leu Thr Gln Ser Pro Ala Ser Leu Ala Val
   1               5                   10                  15
tct cta ggg cag agg gcc acc atc tcc tgc aag gcc agc caa agt gtt     95
Ser Leu Gly Gln Arg Ala Thr Ile Ser Cys Lys Ala Ser Gln Ser Val
              20                  25                  30
gat tat gat ggt gat agt tat atg aac tgg tac caa cag aaa cca gga    143
Asp Tyr Asp Gly Asp Ser Tyr Met Asn Trp Tyr Gln Gln Lys Pro Gly
          35                  40                  45
cag cca ccc aaa ctc ctc atc tat gct gca tcc aat cta gaa tct ggg    191
Gln Pro Pro Lys Leu Leu Ile Tyr Ala Ala Ser Asn Leu Glu Ser Gly
          50                  55                  60
atc cca gcc aga ttt act ggc agt ggg tct ggg aca gac ttc acc ctc    239
Ile Pro Ala Arg Phe Thr Gly Ser Gly Ser Gly Thr Asp Phe Thr Leu
      65                  70                  75
aac atc cat cct gtg gag gag gag gat act gca acc tat tac tgt caa    287
Asn Ile His Pro Val Glu Glu Glu Asp Thr Ala Thr Tyr Tyr Cys Gln
80                  85                  90                  95
caa agt tat gag gat cct ccg aca ttc gct gga ggc acc aac ctg gaa    335
Gln Ser Tyr Glu Asp Pro Pro Thr Phe Ala Gly Gly Thr Asn Leu Glu
              100                 105                 110
atc aag ggt gga ggc ggt tca ggc gga ggt ggc tcc gga ggt ggc gga    383
Ile Lys Gly Gly Gly Gly Ser Gly Gly Gly Gly Ser Gly Gly Gly Gly
          115                 120                 125

tcg atg gtg cat ctt cag gag tcg gaa cct gag ctg gtg aag cct ggg    431
Ser Met Val His Leu Gln Glu Ser Glu Pro Glu Leu Val Lys Pro Gly
          130                 135                 140
gct tca gtg aag atg tcc tgc aag gct tct gga tac aca ttc act gac    479
Ala Ser Val Lys Met Ser Cys Lys Ala Ser Gly Tyr Thr Phe Thr Asp
    145                 150                 155
tat gtt ata aac tgg gtg aag cag aga act gga cag ggc ctt gag tgg    527
Tyr Val Ile Asn Trp Val Lys Gln Arg Thr Gly Gln Gly Leu Glu Trp
160                 165                 170                 175
att gga gag act tat act gga agt ggc agt agt tat tac aat gag aag    575
Ile Gly Glu Thr Tyr Thr Gly Ser Gly Ser Ser Tyr Tyr Asn Glu Lys
              180                 185                 190
ttc aag gac aag gcc aca ctg act gta gac aaa gcc tcc aat ata gcc    623
Phe Lys Asp Lys Ala Thr Leu Thr Val Asp Lys Ala Ser Asn Ile Ala
          195                 200                 205
tac atg cag ctc agc agc ctg aca tct gag gac tct gcg gtc tat ttc    671
Tyr Met Gln Leu Ser Ser Leu Thr Ser Glu Asp Ser Ala Val Tyr Phe
          210                 215                 220
tgt gca aga cgg ggt aaa gga acc ggg ttt gct ttt tgg ggc caa ggg    719
Cys Ala Arg Arg Gly Lys Gly Thr Gly Phe Ala Phe Trp Gly Gln Gly
    225                 230                 235
act ctg gtc act gtc tct gca gcg gcc gc                             748
Thr Leu Val Thr Val Ser Ala Ala Ala
240                 245
```

<210> 3
<211> 248
<212> PRT
<213> mouse and artificial sequence
<220>
<223> Single chain Fv. anti-Leu3a VL and VH fragment was connected with f

lexible linker. Modified with restriction enzyme site.

<400> 3

```
Met Ala Asp Ile Val Leu Thr Gln Ser Pro Ala Ser Leu Ala Val Ser
 1               5                  10              15
Leu Gly Gln Arg Ala Thr Ile Ser Cys Lys Ala Ser Gln Ser Val Asp
            20                  25              30
Tyr Asp Gly Asp Ser Tyr Met Asn Trp Tyr Gln Gln Lys Pro Gly Gln
            35                  40              45
Pro Pro Lys Leu Leu Ile Tyr Ala Ala Ser Asn Leu Glu Ser Gly Ile
        50                  55              60
Pro Ala Arg Phe Thr Gly Ser Gly Ser Gly Thr Asp Phe Thr Leu Asn
65                  70                  75                  80
Ile His Pro Val Glu Glu Glu Asp Thr Ala Thr Tyr Tyr Cys Gln Gln
            85                  90                  95
Ser Tyr Glu Asp Pro Pro Thr Phe Ala Gly Gly Thr Asn Leu Glu Ile
            100                 105                 110
Lys Gly Gly Gly Gly Ser Gly Gly Gly Gly Ser Gly Gly Gly Gly Ser
            115                 120                 125

Met Val His Leu Gln Glu Ser Glu Pro Glu Leu Val Lys Pro Gly Ala
    130                 135                 140
Ser Val Lys Met Ser Cys Lys Ala Ser Gly Tyr Thr Phe Thr Asp Tyr
145                 150                 155                 160
Val Ile Asn Trp Val Lys Gln Arg Thr Gly Gln Gly Leu Glu Trp Ile
            165                 170                 175
Gly Glu Thr Tyr Thr Gly Ser Gly Ser Ser Tyr Tyr Asn Glu Lys Phe
            180                 185                 190
Lys Asp Lys Ala Thr Leu Thr Val Asp Lys Ala Ser Asn Ile Ala Tyr
            195                 200                 205
Met Gln Leu Ser Ser Leu Thr Ser Glu Asp Ser Ala Val Tyr Phe Cys
    210                 215                 220
Ala Arg Arg Gly Lys Gly Thr Gly Phe Ala Phe Trp Gly Gln Gly Thr
225                 230                 235                 240
Leu Val Thr Val Ser Ala Ala Ala
                245
```

<210> 4
<211> 6
<212> PRT
<213> Artificial Sequence
<220>
<223> His tag. Designed peptide for metal Affinity chromatography purific ation.
<400> 4

```
His His His His His His
 1               5
```

<210> 5
<211> 422
<212> DNA
<213> Streptmyces Avidinii and artificial sequence
<220>
<223> Streptavidin core peptide cording sequence. Codon modified Streptav

idin core gene. Modified with restriction enzyme site and His tag

<400> 5

```
      c  tcg agc gcg gaa gcc ggc att acc ggc acc tgg tat aac cag ctg        46
         Ser Ser Ala Glu Ala Gly Ile Thr Gly Thr Trp Tyr Asn Gln Leu
          1               5                  10                  15
        ggc agc acc ttc atc gtc acc gcg ggt gcg gac ggc gcg ctg acc ggc      94
        Gly Ser Thr Phe Ile Val Thr Ala Gly Ala Asp Gly Ala Leu Thr Gly
                         20                  25                  30
        acc tat gaa agc gcg gtg ggc aac gcg gaa tct cgt tat gtg ctg acc     142
        Thr Tyr Glu Ser Ala Val Gly Asn Ala Glu Ser Arg Tyr Val Leu Thr
                     35                  40                  45
        ggt cgt tat gat tcg gcg ccg gcc acc gac ggt agt ggc acc gct ctg     190
        Gly Arg Tyr Asp Ser Ala Pro Ala Thr Asp Gly Ser Gly Thr Ala Leu
                 50                  55                  60
        ggc tgg acc gtg gcg tgg aaa aat aat tac cgt aat gcg cat tct gcg     238
        Gly Trp Thr Val Ala Trp Lys Asn Asn Tyr Arg Asn Ala His Ser Ala


             65                  70                  75
        acc acg tgg agc ggc cag tat gtg ggc ggc gcg gaa gcg cgt atc aac     286
        Thr Thr Trp Ser Gly Gln Tyr Val Gly Gly Ala Glu Ala Arg Ile Asn
         80                  85                  90                  95
        acg cag tgg ctg ctg acc tcc ggc acc acc gaa gcg aac gct tgg aaa     334
        Thr Gln Trp Leu Leu Thr Ser Gly Thr Thr Glu Ala Asn Ala Trp Lys
                         100                 105                 110
        agc acc ctg gtg ggc cat gac acg ttt acc aaa gtc aaa ccg tcg gcg     382
        Ser Thr Leu Val Gly His Asp Thr Phe Thr Lys Val Lys Pro Ser Ala
                         115                 120                 125
        gcg agc ggg tcg acc cac cat cat cat cac cac taa gct t               422
        Ala Ser Gly Ser Thr His His His His His His
        130 135
```

<210> 6
<211> 138
<212> PRT
<213> Streptmyces Avidinii and artificial sequence
<220>
<223> Streptavidin core peptide cording sequence. Codon modified Streptav
idin core gene. Modified with restriction enzyme site and His tag
<400> 6

```
Ser Ser Ala Glu Ala Gly Ile Thr Gly Thr Trp Tyr Asn Gln Leu Gly
1               5                   10                  15
Ser Thr Phe Ile Val Thr Ala Gly Ala Asp Gly Ala Leu Thr Gly Thr
            20              25              30
Tyr Glu Ser Ala Val Gly Asn Ala Glu Ser Arg Tyr Val Leu Thr Gly
        35                  40                  45
Arg Tyr Asp Ser Ala Pro Ala Thr Asp Gly Ser Gly Thr Ala Leu Gly
    50              55                  60
Trp Thr Val Ala Trp Lys Asn Asn Tyr Arg Asn Ala His Ser Ala Thr
65              70                  75                  80
Thr Trp Ser Gly Gln Tyr Val Gly Gly Ala Glu Ala Arg Ile Asn Thr
            85                  90                  95
Gln Trp Leu Leu Thr Ser Gly Thr Thr Glu Ala Asn Ala Trp Lys Ser
        100                 105                 110
Thr Leu Val Gly His Asp Thr Phe Thr Lys Val Lys Pro Ser Ala Ala
        115                 120                 125
Ser Gly Ser Thr His His His His His His
        130                 135
```

<210>7
<211> 8
<212> PRT
<213> Artificial Sequence
<220>
<223> FLAG tag. Designed peptide for anti-FLAG antibody detection
<400> 7

```
Asp Tyr Lys Asp Asp Asp Asp Lys
1               5
```

<210> 8
<211> 41
<212> DNA
<213> Artificial Sequence
<220>
<223> FLAG tag. Designed peptide for spacer between ligand and domain
<400> 8

```
gcg gcc gca gac tac aag gat gac gat gac aaa ggc tcg ag      41
Ala Ala Ala Asp Tyr Lys Asp Asp Asp Asp Lys Gly Ser
1               5                   10
```

<210> 9
<211> 13
<212> PRT
<213> Artificial Sequence
<220>
<223> FLAG tag. Designed peptide for spacer between ligand and domain
<400> 9

```
Ala Ala Ala Asp Tyr Lys Asp Asp Asp Asp Lys Gly Ser
1               5                   10
```

<210> 10

29

<211> 10
<212> PRT
<213> Artificial Sequence
<220>
<223> c-myc epitope tag. Designed peptide for anti-c-myc antibody detecti
on
<400> 10

```
                              Glu Gln Lys Leu Ile Ser Glu Glu Asp Leu
                               1               5                  10
```

<210> 11
<211> 127
<212> DNA
<213> Artificial Sequence
<220>
<223> FLAG tag, c-myc epitope tag, His tag. Designed peptide for purifica
tion and control experiment. Modified with restriction enzyme site
<400> 11

```
        gcg gcc gca gac tac aag gat gac gat gac aaa ggc tcg agc gag cag          48
        Ala Ala Ala Asp Tyr Lys Asp Asp Asp Asp Lys Gly Ser Ser Glu Gln
         1               5                  10                  15
        aag ctg atc agc gaa gag gat ctg ggc tcg agg tcg acc cac cat cat          96
        Lys Leu Ile Ser Glu Glu Asp Leu Gly Ser Arg Ser Thr His His His
                         20                  25                  30
        cat cac cac ggg tcg acc aaa tga taa gct t                               127
        His His His Gly Ser Thr Lys Stop
                         35
```

<210> 12
<211> 39
<212> PRT
<213> Artificial Sequence
<220>
<223> FLAG tag, c-myc epitope tag, His tag. Designed peptide for purifica
tion and control experiment. Modified with restriction enzyme site
<400> 12

```
        Ala Ala Ala Asp Tyr Lys Asp Asp Asp Asp Lys Gly Ser Ser Glu Gln
         1               5                  10                  15
        Lys Leu Ile Ser Glu Glu Asp Leu Gly Ser Arg Ser Thr His His His
                         20                  25                  30
        His His His Gly Ser Thr Lys
                         35
```

<210> 13
<211> 760
<212> DNA
<213> mouse and artificial sequence
<220>
<223> Single chain Fv. 4G7 antibody VL and VH fragnent was connected with
flexible linker. Modified with restriction enzyme site.
<400> 13

```
         cc atg gcc gat att gtg atg act cag gct gca ccc tct ata cct gtc       47
            Met Ala Asp Ile Val Met Thr Gln Ala Ala Pro Ser Ile Pro Val
             1               5              10              15
         act cct gga gag tca gta tcc atc tcc tgc agg tct agt aag agt ctc       95
         Thr Pro Gly Glu Ser Val Ser Ile Ser Cys Arg Ser Ser Lys Ser Leu
                         20                  25                  30
         ctg aat agt aat ggc aac act tac ttg tat tgg ttc ctg cag agg cca      143
         Leu Asn Ser Asn Gly Asn Thr Tyr Leu Tyr Trp Phe Leu Gln Arg Pro
                     35                  40                  45
         ggc cag tct cct cag ctc ctg ata tat cgg atg tcc aac ctt gcc tca      191
         Gly Gln Ser Pro Gln Leu Leu Ile Tyr Arg Met Ser Asn Leu Ala Ser
                 50                  55                  60
         gga gtc cca gac agg ttc agt ggc agt ggg tca gga act gct ttc aca      239
         Gly Val Pro Asp Arg Phe Ser Gly Ser Gly Ser Gly Thr Ala Phe Thr
                 65                  70                  75
         ctg aga atc agt aga gtg gag gct gag gat gtg ggt gtt tat tac tgt      287
         Leu Arg Ile Ser Arg Val Glu Ala Glu Asp Val Gly Val Tyr Tyr Cys
         80                  85                  90                  95
         atg caa cat cta gaa tat ccg ttc acg ttc ggt gct ggg acc aag ctg      335
         Met Gln His Leu Glu Tyr Pro Phe Thr Phe Gly Ala Gly Thr Lys Leu
                         100                 105                 110
         gag ctg aaa ggt gga ggc ggt tca ggc gga ggt ggc tcc gga ggt ggc      383
         Glu Leu Lys Gly Gly Gly Gly Ser Gly Gly Gly Gly Ser Gly Gly Gly
                         115                 120                 125
         gga tcg gag gtc cag ctg cag cag tct gga cct gag ctg ata aag cct      431
         Gly Ser Glu Val Gln Leu Gln Gln Ser Gly Pro Glu Leu Ile Lys Pro
                         130                 135                 140
         ggg gct tca gtg aag atg tcc tgc aag gct tct gga tac aca ttc act      479



         Gly Ala Ser Val Lys Met Ser Cys Lys Ala Ser Gly Tyr Thr Phe Thr
                 145                 150                 155
         agc tat gtt atg cac tgg gtg aag cag aag cct ggg cag ggc ctt gag      527
         Ser Tyr Val Met His Trp Val Lys Gln Lys Pro Gly Gln Gly Leu Glu
         160                 165                 170                 175
         tgg att gga tat att aat cct tac aat gat ggt act aag tac aat gag      575
         Trp Ile Gly Tyr Ile Asn Pro Tyr Asn Asp Gly Thr Lys Tyr Asn Glu
                         180                 185                 190
         aag ttc aaa ggc aag gcc aca ctg act tca gac aaa tcc tcc agc aca      623
         Lys Phe Lys Gly Lys Ala Thr Leu Thr Ser Asp Lys Ser Ser Ser Thr
                     195                 200                 205
         gcc tac atg gag ctc agc agc ctg acc tct gag gac tct gcg gtc tat      671
         Ala Tyr Met Glu Leu Ser Ser Leu Thr Ser Glu Asp Ser Ala Val Tyr
                 210                 215                 220
         tac tgt gca aga ggg act tat tac tac ggt agt agg gta ttt gac tac      719
         Tyr Cys Ala Arg Gly Thr Tyr Tyr Tyr Gly Ser Arg Val Phe Asp Tyr
                 225                 230                 235
         tgg ggc caa ggc acc act ctc aca gtc tcc tca gcg gcc gc              760
         Trp Gly Gln Gly Thr Thr Leu Thr Val Ser Ser Ala Ala
         240                 245                 250
```

<210> 14
<211> 252
<212> PRT
<213> mouse and artificial sequence
<220>

<223> Single chain Fv. 4G7 antibody VL and VH fragment was connected with flexible linker. Modified with restriction enzyme site.
<400> 14

```
Met Ala Asp Ile Val Met Thr Gln Ala Ala Pro Ser Ile Pro Val Thr
1               5               10              15
Pro Gly Glu Ser Val Ser Ile Ser Cys Arg Ser Ser Lys Ser Leu Leu
            20              25              30
Asn Ser Asn Gly Asn Thr Tyr Leu Tyr Trp Phe Leu Gln Arg Pro Gly
        35              40              45
Gln Ser Pro Gln Leu Leu Ile Tyr Arg Met Ser Asn Leu Ala Ser Gly
        50              55              60
Val Pro Asp Arg Phe Ser Gly Ser Gly Ser Gly Thr Ala Phe Thr Leu
65              70              75                      80
Arg Ile Ser Arg Val Glu Ala Glu Asp Val Gly Val Tyr Tyr Cys Met
                85              90              95
Gln His Leu Glu Tyr Pro Phe Thr Phe Gly Ala Gly Thr Lys Leu Glu
        100             105             110
Leu Lys Gly Gly Gly Gly Ser Gly Gly Gly Gly Ser Gly Gly Gly Gly
        115             120             125
Ser Glu Val Gln Leu Gln Gln Ser Gly Pro Glu Leu Ile Lys Pro Gly
    130             135             140
Ala Ser Val Lys Met Ser Cys Lys Ala Ser Gly Tyr Thr Phe Thr Ser
145             150             155             160

Tyr Val Met His Trp Val Lys Gln Lys Pro Gly Gln Gly Leu Glu Trp
            165             170             175
Ile Gly Tyr Ile Asn Pro Tyr Asn Asp Gly Thr Lys Tyr Asn Glu Lys
            180             185             190
Phe Lys Gly Lys Ala Thr Leu Thr Ser Asp Lys Ser Ser Ser Thr Ala
        195             200             205
Tyr Met Glu Leu Ser Ser Leu Thr Ser Glu Asp Ser Ala Val Tyr Tyr
    210             215             220
Cys Ala Arg Gly Thr Tyr Tyr Tyr Gly Ser Arg Val Phe Asp Tyr Trp
225             230             235             240
Gly Gln Gly Thr Thr Leu Thr Val Ser Ser Ala Ala
            245             250
```

**Claims**

1. A substrate for biological fluid treatment in which an assembly of recognition molecules having a selective binding property for at least one target substance which is selected from a biorelated substance, a bacterium, a cell, a cell aggregation, a virus, or a virus-infected cell, is covalently immobilized on a surface of the substrate,
   wherein each recognition molecule is a linear molecule comprising a ligand moiety which selectively binds to the target substance, and an association domain,
   wherein four recognition molecules form the assembly structure through association of their association domains,
   wherein the assembly is covalently bound to the substrate via the ligand moieties of the recognition molecules in such a way that at least one ligand moiety remains externally oriented with respect to the substrate surface,
   wherein each recognition molecule is a single-chain polypeptide obtained by expression of a nucleic acid in which a nucleic acid that encodes the ligand moiety for constituting the recognition molecule and a nucleic acid that encodes the association domain are ligated, and
   wherein the association domain is a polypeptide region translated from a nucleic acid that encodes a full length, or a part of, streptavidin or avidin.

2. The substrate for biological fluid treatment according to claim 1, wherein the assembly of recognition molecules is directly bound to the substrate surface or via active groups.

3. The substrate for biological fluid treatment according to claim 2, wherein the active group is an epoxy group obtained through activation of hydroxyl groups and amino groups on the substrate surface using epichlorhydrin.

4. The substrate for biological fluid treatment according to claim 2, wherein the active group is introduced into the substrate surface by means of graft polymerization, plasma treatment, corona treatment, chemical treatment, or exposure to gas.

5. The substrate according to claim 1, wherein the polypeptide is obtained by expression of a nucleic acid in which a nucleic acid that encodes the ligand moiety for constituting the recognition molecule and a nucleic acid that encodes the association domain are ligated by a nucleic acid that encodes a spacer, and the spacer is a peptidic spacer composed of 0 to 100 amino acids.

6. The substrate for biological fluid treatment according to claim 1 or 5, wherein the polypeptide to be translated from the nucleic acid that encodes the ligand moiety has a molecular weight of 100 kD or less.

7. The substrate for biological fluid treatment according to any one of claims 1, 5 or 6, wherein the polypeptide to be translated from the nucleic acid that encodes the association domain has a molecular weight of 50 kD or less.

8. The substrate for biological fluid treatment according to any one of claims 1 or 5 to 4, wherein the polypeptide to be translated from the nucleic acid that encodes the ligand moiety is a single-chain antibody.

9. The substrate for biological fluid treatment according to any one of claims 1, or 5 to 8, wherein the molecular weight ratio of the polypeptide that constitutes the ligand moiety to the polypeptide that constitutes the association domain is within a range of 1:10 to 20:1.

10. The substrate for biological fluid treatment according to any one of claims 1 or 5 to 9, which is produced by:

   - translating a nucleic acid that encodes a recognition molecule having a selective binding property for the target substance, into a polypeptide;
   - denaturing/solubilizing the recognition molecule polypeptide that has been insolubilized during the process of said translation, with an aqueous solution containing guanidine hydrochloride or urea; and
   - subsequently renaturating the three-dimensional structure of the recognition molecule polypeptide by removing guanidine hydrochloride or urea.

11. The substrate for biological fluid treatment according to any one of claims 1 or 5 to 10, which is produced by:

   - introducing an active group for forming a covalent bond with a recognition molecule having a selective binding property for the target substance into a surface of a water-insoluble substrate; and
   - covalently immobilizing the recognition molecule onto the substrate surface by contacting the water-insoluble substrate with an aqueous solution containing the recognition molecule at a temperature of 0°C to 50°C for 10 or more seconds.

12. A method for production of the substrate for biological fluid treatment according to any one of claims 1 or 5 to 11, which comprises:

   (i) a step of translating a nucleic acid that encodes a recognition molecule having a selective binding property for at least one target substance which is selected from a biorelated substance, a bacterium, a cell, a cell aggregation, a virus, or a virus-infected cell, into a polypeptide;
   (ii) a step of denaturing/solubilizing the recognition molecule polypeptide that has been insolubilized during the process of said translation, with an aqueous solution containing guanidine hydrochloride or urea; and
   (iii) a step of, after the step (ii), renaturating the three-dimensional structure of the recognition molecule polypeptide by removing guanidine hydrochloride or urea.

13. A method for production of the substrate for biological fluid treatment according to any one of claims 1 or 5 to 12, which comprises:

(i) a step of introducing an active group into a surface of a water-insoluble substrate for forming a covalent bond with a recognition molecule having a selective binding property for at least one target substance which is selected from a biorelated substance, a bacterium, a cell, a cell aggregation, a virus, or a virus-infected cell; and
(ii) a step of covalently immobilizing the recognition molecule onto the substrate surface by contacting the water-insoluble substrate with an aqueous solution containing the recognition molecule at a temperature of 0°C to 50 °C for 10 or more seconds.

**14.** A device for biological fluid treatment which is obtained by loading the substrate for biological fluid treatment according to any one of claims 1 to 11 in a container having an inlet and an outlet.

**15.** The device for biological fluid treatment according to claim 14 which is used for the treatment of a disease.

**16.** A method for production of the device for biological fluid treatment according to claim 14 or 15, which comprises a step of loading the substrate for biological fluid treatment according to any one of claims 1 to 11 in a container having an inlet and an outlet.

**17.** A method for treatment of a biological fluid, which comprises a step of flowing an aqueous solution containing a target substance through the device for biological fluid treatment according to claim 14 or 15, wherein methods for the treatment of the human or animal body by surgery or therapy are excluded.

**18.** A method for treatment of blood, which comprises a step of flowing blood containing a target substance through the device for biological fluid treatment according to claim 14 or 15, wherein methods for the treatment of the human or animal body by surgery or therapy are excluded.

**Patentansprüche**

**1.** Substrat zur Behandlung einer biologischen Flüssigkeit, bei dem eine Anordnung von Erkennungsmolekülen mit einer selektiven Bindungseigenschaft für wenigstens eine Targetsubstanz, die aus einer bioartigen Substanz, einem Bakterium, einer Zelle, einem Zellaggregat, einem Virus oder einer virusinfizierten Zelle ausgewählt ist, kovalent auf einer Fläche des Substrats immobilisiert sind,
wobei jedes Erkennungsmolekül ein lineares Molekül ist, das eine Ligandeneinheit, die selektiv an die Targetsubstanz bindet, und eine Assoziationsdomäne umfasst,
wobei vier Erkennungsmoleküle durch Assoziation ihrer Assoziationsdomänen die Anordnungsstruktur bilden,
wobei die Anordnung über die Ligandeneinheiten der Erkennungsmoleküle in einer solchen Weise kovalent an das Substrat gebunden ist, dass wenigstens eine Ligandeneinheit in Bezug auf die Substratoberfläche nach außen gerichtet bleibt,
wobei jedes Erkennungsmolekül ein einzelkettiges Polypeptid ist, das durch Expression einer Nucleinsäure erhalten wird, wobei eine Nucleinsäure, die die Ligandeneinheit zum Aufbau des Erkennungsmoleküls codiert, und eine Nucleinsäure, die die Assoziationsdomäne codiert, miteinander verknüpft sind, und
wobei die Assoziationsdomäne ein Polypeptidbereich ist, der ausgehend von einer Nucleinsäure translatiert wird, die die volle Länge oder einen Teil von Streptavidin oder Avidin codiert.

**2.** Substrat zur Behandlung einer biologischen Flüssigkeit gemäß Anspruch 1, wobei die Anordnung von Erkennungsmolekülen direkt oder über aktive Gruppen an die Substratoberfläche gebunden ist.

**3.** Substrat zur Behandlung einer biologischen Flüssigkeit gemäß Anspruch 2, wobei die aktive Gruppe eine Epoxidgruppe ist, die durch Aktivierung von Hydroxygruppen und Aminogruppen auf der Substratoberfläche mit Hilfe von Epichlorhydrin erhalten wird.

**4.** Substrat zur Behandlung einer biologischen Flüssigkeit gemäß Anspruch 2, wobei die aktive Gruppe mittels Pfropfpolymerisation, Plasmabehandlung, Koronabehandlung, chemischer Behandlung oder Einwirkung von Gas in die Substratoberfläche eingeführt wird.

**5.** Substrat gemäß Anspruch 1, wobei das Polypeptid durch Expression einer Nucleinsäure erhalten wird, wobei eine Nucleinsäure, die die Ligandeneinheit zum Aufbau des Erkennungsmoleküls codiert, und eine Nucleinsäure, die die Assoziationsdomäne codiert, über eine Nucleinsäure, die einen Spacer codiert, miteinander verknüpft sind und der Spacer ein peptidischer Spacer ist, der aus 0 bis 100 Aminosäuren besteht.

34

6. Substrat zur Behandlung einer biologischen Flüssigkeit gemäß Anspruch 1 oder 5, wobei das Polypeptid, das ausgehend von der Nucleinsäure, die die Ligandeneinheit codiert, translatiert werden soll, ein Molekulargewicht von 100 kD oder weniger aufweist.

7. Substrat zur Behandlung einer biologischen Flüssigkeit gemäß einem der Ansprüche 1, 5 oder 6, wobei das Polypeptid, das ausgehend von der Nucleinsäure, die die Assoziationsdomäne codiert, translatiert werden soll, ein Molekulargewicht von 50 kD oder weniger aufweist.

8. Substrat zur Behandlung einer biologischen Flüssigkeit gemäß einem der Ansprüche 1 oder 5 bis 4, wobei das Polypeptid, das ausgehend von der Nucleinsäure, die die Ligandeneinheit codiert, translatiert werden soll, ein einzelkettiger Antikörper ist.

9. Substrat zur Behandlung einer biologischen Flüssigkeit gemäß einem der Ansprüche 1 oder 5 bis 8, wobei das Molekulargewichtsverhältnis des Polypeptids, das die Ligandeneinheit bildet, zu dem Polypeptid, das die Assoziationsdomäne bildet, im Bereich von 1:10 bis 20:1 liegt.

10. Substrat zur Behandlung einer biologischen Flüssigkeit gemäß einem der Ansprüche 1 oder 5 bis 9, das hergestellt wird durch:

   - Translatieren einer Nucleinsäure, die ein Erkennungsmolekül mit einer selektiven Bindungseigenschaft für die Targetsubstanz codiert, zu einem Polypeptid;
   - Denaturieren/Solubilisieren des Erkennungsmolekül-Polypeptids, das während des Vorgangs der Translation insolubilisiert wurde, mit einer wässrigen Lösung, die Guanidin-Hydrochlorid oder Harnstoff enthält; und
   - anschließend Renaturieren der dreidimensionalen Struktur des Erkennungsmolekül-Polypeptids durch Entfernen des Guanidin-Hydrochlorids oder des Harnstoffs.

11. Substrat zur Behandlung einer biologischen Flüssigkeit gemäß einem der Ansprüche 1 oder 5 bis 10, das hergestellt wird durch:

   - Einführen einer aktiven Gruppe zur Bildung einer kovalenten Bindung mit einem Erkennungsmolekül, das eine selektive Bindungseigenschaft bezüglich der Targetsubstanz aufweist, in eine Fläche eines wasserunlöslichen Substrats; und
   - kovalentes Immobilisieren des Erkennungsmoleküls auf der Substratoberfläche durch In-Kontakt-Bringen des wasserunlöslichen Substrats mit einer wässrigen Lösung, die das Erkennungsmolekül enthält, bei einer Temperatur von 0 °C bis 50 °C während 10 oder mehr Sekunden.

12. Verfahren zur Herstellung des Substrats zur Behandlung einer biologischen Flüssigkeit gemäß einem der Ansprüche 1 oder 5 bis 11, umfassend:

   (i) einen Schritt des Translatierens einer Nucleinsäure, die ein Erkennungsmolekül codiert, das eine selektive Bindungseigenschaft bezüglich wenigstens einer Targetsubstanz aufweist, die aus einer bioartigen Substanz, einem Bakterium, einer Zelle, einem Zellaggregat, einem Virus oder einer virusinfizierten Zelle ausgewählt ist, zu einem Polypeptid;
   (ii) einen Schritt des Denaturierens/Solubilisierens des Erkennungsmolekül-Polypeptids, das während des Vorgangs der Translation insolubilisiert wurde, mit einer wässrigen Lösung, die Guanidin-Hydrochlorid oder Harnstoff enthält; und
   (iii) einen Schritt des Renaturierens der dreidimensionalen Struktur des Erkennungsmolekül-Polypeptids durch Entfernen des Guanidin-Hydrochlorids oder des Harnstoffs nach Schritt (ii).

13. Verfahren zur Herstellung des Substrats zur Behandlung einer biologischen Flüssigkeit gemäß einem der Ansprüche 1 oder 5 bis 12, umfassend:

   (i) einen Schritt des Einführen einer aktiven Gruppe in eine Fläche eines wasserunlöslichen Substrats zur Bildung einer kovalenten Bindung mit einem Erkennungsmolekül, das eine selektive Bindungseigenschaft bezüglich wenigstens einer Targetsubstanz aufweist, die aus einer bioartigen Substanz, einem Bakterium, einer Zelle, einem Zellaggregat, einem Virus oder einer virusinfizierten Zelle ausgewählt ist; und
   (ii) einen Schritt des kovalenten Immobilisierens des Erkennungsmoleküls auf der Substratoberfläche durch In-Kontakt-Bringen des wasserunlöslichen Substrats mit einer wässrigen Lösung, die das Erkennungsmolekül enthält, bei einer Temperatur von 0 °C bis 50 °C während 10 oder mehr Sekunden.

14. Vorrichtung zur Behandlung einer biologischen Flüssigkeit, die dadurch erhalten wird, dass man das Substrat zur Behandlung einer biologischen Flüssigkeit gemäß einem der Ansprüche 1 bis 11 in einen Behälter einführt, der einen Einlass und einen Auslass aufweist.

15. Vorrichtung zur Behandlung einer biologischen Flüssigkeit gemäß Anspruch 14, die zur Behandlung einer Erkrankung verwendet wird.

16. Verfahren zur Herstellung der Vorrichtung zur Behandlung einer biologischen Flüssigkeit gemäß Anspruch 14 oder 15, umfassend einen Schritt des Einführens des Substrats zur Behandlung einer biologischen Flüssigkeit gemäß einem der Ansprüche 1 bis 11 in einen Behälter, der einen Einlass und einen Auslass aufweist.

17. Verfahren zur Behandlung einer biologischen Flüssigkeit, umfassend einen Schritt des Fließenlassens einer wässrigen Lösung, die eine Targetsubstanz enthält, durch die Vorrichtung zur Behandlung einer biologischen Flüssigkeit gemäß Anspruch 14 oder 15, wobei Verfahren zur Behandlung des menschlichen oder tierischen Körpers durch Chirurgie oder Therapie ausgeschlossen sind.

18. Verfahren zur Behandlung von Blut, umfassend einen Schritt des Fließenlassens von Blut, das eine Targetsubstanz enthält, durch die Vorrichtung zur Behandlung einer biologischen Flüssigkeit gemäß Anspruch 14 oder 15, wobei Verfahren zur Behandlung des menschlichen oder tierischen Körpers durch Chirurgie oder Therapie ausgeschlossen sind.

**Revendications**

1. Substrat pour le traitement d'un liquide biologique dans lequel un ensemble de molécules de reconnaissance ayant une propriété de liaison sélective à l'égard d'au moins une substance cible qui est choisie parmi une substance bioapparentée, une bactérie, une cellule, une agrégation de cellules, un virus, ou une cellule infectée par un virus, est immobilisé par covalence sur une surface du substrat,
dans lequel chaque molécule de reconnaissance est une molécule linéaire comprenant une entité de ligand qui se lie sélectivement à la substance cible, et un domaine d'association,
dans lequel quatre molécules de reconnaissance forment la structure de l'ensemble par l'association de leur domaine d'association,
dans lequel l'ensemble est lié par covalence au substrat par l'intermédiaire des entités de ligand des molécules de reconnaissance de telle manière qu'au moins une entité de ligand demeure orientée vers l'extérieur par rapport à la surface du substrat,
dans lequel chaque molécule de reconnaissance est un polypeptide à chaîne unique obtenu par l'expression d'un acide nucléique, dans lequel un acide nucléique qui code pour l'entité de ligand afin de constituer la molécule de reconnaissance et un acide nucléique qui code pour le domaine d'association, sont ligaturés, et
dans lequel le domaine d'association est une région polypeptidique traduite à partir d'un acide nucléique qui code pour une streptavidine ou une avidine entière ou partielle.

2. Substrat pour le traitement d'un liquide biologique selon la revendication 1, dans lequel l'ensemble de molécules de reconnaissance est directement lié à la surface du substrat ou par l'intermédiaire de groupes actifs.

3. Substrat pour le traitement d'un liquide biologique selon la revendication 2, dans lequel le groupe actif est un groupe époxy obtenu par l'activation de groupes hydroxyle et de groupes amino à la surface du substrat au moyen d'épichlorhydrine.

4. Substrat pour le traitement d'un liquide biologique selon la revendication 2, dans lequel le groupe actif est introduit dans la surface du substrat au moyen d'une polymérisation par greffage, d'un traitement au plasma, d'un traitement corona, d'un traitement chimique, ou de d'une exposition à un gaz.

5. Substrat selon la revendication 1, dans lequel le polypeptide est obtenu par l'expression d'un acide nucléique, dans lequel un acide nucléique qui code pour l'entité de ligand afin de constituer la molécule de reconnaissance et un acide nucléique qui code pour le domaine d'association, sont ligaturés par un acide nucléique qui code pour un espaceur, et l'espaceur est un espaceur peptidique composé de 0 à 100 acides aminés.

**EP 2 036 584 B1**

6. Substrat pour le traitement d'un liquide biologique selon la revendication 1 ou 5, dans lequel le polypeptide à traduire à partir de l'acide nucléique qui code pour l'entité de ligand a une masse moléculaire de 100 kD ou moins.

7. Substrat pour le traitement d'un liquide biologique selon l'une quelconque des revendications 1, 5 ou 6, dans lequel le polypeptide à traduire à partir de l'acide nucléique qui code pour le domaine d'association a une masse moléculaire de 50 kD ou moins.

8. Substrat pour le traitement d'un liquide biologique selon l'une quelconque des revendications 1 ou 5 à 4, dans lequel le polypeptide à traduire à partir de l'acide nucléique qui code pour l'entité de ligand est un anticorps à chaîne unique.

9. Substrat pour le traitement d'un liquide biologique selon l'une quelconque des revendications 1, ou 5 à 8, dans lequel le rapport de la masse moléculaire du polypeptide qui constitue l'entité de ligand à celle du polypeptide qui constitue le domaine d'association est dans une plage de 1/10 à 20/1.

10. Substrat pour le traitement d'un liquide biologique selon l'une quelconque des revendications 1 ou 5 à 9, qui est produit par :

- la traduction d'un acide nucléique qui code pour une molécule de reconnaissance ayant une propriété de liaison sélective à l'égard de la substance cible, en un polypeptide ;
- la dénaturation/solubilisation du polypeptide de la molécule de reconnaissance qui a été rendu insoluble au cours du processus de ladite traduction, au moyen d'une solution aqueuse contenant du chlorhydrate de guanidine ou de l'urée ; et
- par la suite, par la renaturation de la structure tridimensionnelle du polypeptide de la molécule de reconnaissance par séparation du chlorhydrate de guanidine ou de l'urée.

11. Substrat pour le traitement d'un liquide biologique selon l'une quelconque des revendications 1 ou 5 à 10, qui est produit par :

- l'introduction d'un groupe actif pour former une liaison covalente avec une molécule de reconnaissance ayant une propriété de liaison sélective à l'égard de la substance cible dans une surface d'un substrat insoluble dans l'eau ; et par
- l'immobilisation, par covalence, de la molécule de reconnaissance à la surface du substrat par la mise en contact du substrat insoluble dans l'eau avec une solution aqueuse contenant la molécule de reconnaissance à une température de 0 °C à 50 °C pendant 10 secondes ou plus.

12. Procédé de production du substrat pour le traitement d'un liquide biologique selon l'une quelconque des revendications 1 ou 5 à 11, qui comprend :

(i) une étape de traduction d'un acide nucléique qui code pour une molécule de reconnaissance ayant une propriété de liaison sélective à l'égard d'au moins une substance cible qui est choisie parmi une substance bioapparentée, une bactérie, une cellule, une agrégation de cellules, un virus, ou une cellule infectée par un virus, en un polypeptide ;
(ii) une étape de dénaturation/solubilisation du polypeptide de la molécule de reconnaissance qui a été rendu insoluble au cours du processus de ladite traduction, au moyen d'une solution aqueuse contenant du chlorhydrate de guanidine ou de l'urée; et
(iii) une étape, après l'étape (ii), de renaturation de la structure tridimensionnelle du polypeptide de la molécule de reconnaissance par séparation du chlorhydrate de guanidine ou de l'urée.

13. Procédé de production du substrat pour le traitement d'un liquide biologique selon l'une quelconque des revendications 1 ou 5 à 12, qui comprend :

(i) une étape d'introduction d'un groupe actif dans une surface d'un substrat insoluble dans l'eau pour former une liaison covalente avec une molécule de reconnaissance ayant une propriété de liaison sélective à l'égard d'au moins une substance cible qui est choisie parmi une substance bioapparentée, une bactérie, une cellule, une agrégation de cellules, un virus, ou une cellule infectée par un virus ; et
(ii) une étape d'immobilisation, par covalence, de la molécule de reconnaissance à la surface du substrat par la mise en contact du substrat insoluble dans l'eau avec une solution aqueuse contenant la molécule de reconnaissance à une température de 0 °C à 50 °C pendant 10 secondes ou plus.

**14.** Dispositif de traitement d'un liquide biologique que l'on obtient en chargeant le substrat pour le traitement d'un liquide biologique selon l'une quelconque des revendications 1 à 11 dans un récipient ayant une entrée et une sortie.

**15.** Dispositif de traitement d'un liquide biologique selon la revendication 14 que l'on utilise pour le traitement d'une maladie.

**16.** Procédé de production du dispositif de traitement d'un liquide biologique selon la revendication 14 ou 15, qui comprend une étape de chargement du substrat pour le traitement d'un liquide biologique selon l'une quelconque des revendications 1 à 11 dans un récipient ayant une entrée et une sortie.

**17.** Procédé de traitement d'un liquide biologique, qui comprend une étape consistant à faire passer une solution aqueuse contenant une substance cible à travers le dispositif de traitement d'un liquide biologique selon la revendication 14 ou 15, les procédés de traitement de l'organisme humain ou animal par chirurgie ou thérapie étant exclus.

**18.** Procédé de traitement du sang, qui comprend une étape d'écoulement du sang contenant une substance cible à travers le dispositif de traitement d'un liquide biologique selon la revendication 14 ou 15, les procédés de traitement de l'organisme humain ou animal par chirurgie ou thérapie étant exclus.

[Fig.1]

heat treatment

Biotin addition

tetramer via domein region (SA)

(stable if treated with heat in the presence of biotin)

220kD

97.4kD

66kD

46kD ← monomer

30kD

21.5kD

14.3kD

[Fig.2]

ligand site (single chain antibody)

tetramer formation

spacer

association domain (streptavidin)

[Fig.3]

ligand sites which were externally oriented

substrate surface

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI332680 B
- JP HEI6154316 B
- JP HEI6154317 B
- JP HEI6218051 B
- JP HEI6269663 B
- JP HEI11332594 B
- JP 2000217909 A
- JP 2003052817 A
- JP 2003501096 A
- WO 00075333 PCT
- WO 2006107617 PCT
- WO 9925463 A
- JP 2000093172 A

**Non-patent literature cited in the description**

- *Applied and Environmental Microbiology,* July 1998, 2497-2502
- *Blood,* 2004, vol. 104, 227-236
- *Clinical Cancer Research,* vol. 6, 406-414
- *Protein Engineering,* 1996, vol. 9 (2), 203-211
- *Human Antibodies Hybridomas,* 1995, vol. 6 (3), 93-101
- *Foodborne Pathogens and Disease,* 2007, vol. 4 (1), 74-83
- **DUBEL et al.** preparation of a fusion protein comprising a single chain antibody (scFv), a core-streptavidin, a c-myc tag, an extra free cysteine, and a His-tag These fusion proteins assemble into tetramers. *J. Immunol. Meth.,* 1995, vol. 178, 201-209
- **GROVENDER et al.** *Kidney Int.,* 2004, vol. 65, 310-322
- **K. NORD ; O. NORD ; M. UHLEN et al.** *Eur. J. Biochem,* 2001, vol. 268, 4269
- **I. S. KIM ; J. H. SHIM ; Y. T. SUH et al.** *Biosci. Biotechnol. Biochem.,* 2002, vol. 66, 1148
- **V. BATORI ; A. KOIDE ; S. KOIDE.** *Protein Eng.,* 2002, vol. 15, 1015
- **E. LUNDE ; V. LAUVRAK ; I. B. RASMUSSEN et al.** *Biochem. Soc. Trans,* 2002, vol. 30, 500
- **A. MURUGANANDAM ; J. TANHA ; S. NARANG ; D. STANIMIROVIC.** *The FASEB J.,* 2002, vol. 16, 240
- **B. WLOTZKA ; S. LEVA ; B. ESCHGFALLER et al.** *Proc. Natl. Acad. Sci. USA,* 2002, vol. 99, 8898
- **J WILLUDA et al.** *Journal of Biological Chemistry,* 2001, vol. 276, 14385
- **M RHEINNECKER et al.** *The Journal of Immunology,* 1996, vol. 157, 2989
- **M. NISHIKATA ; K. KASAI ; S. ISHII.** *J. Biochem.,* 1977, vol. 82, 1475
- *Proc. Natl. Acad. Sci. USA,* 1995, vol. 92, 3180-3184
- *Journal of Applied Microbiology,* February 2007, vol. 102, 303-312